# EUROPÄISCHE PATENTSCHRIFT

(11) **EP 1 979 464 B1**
(45) Veröffentlichungstag und Bekanntmachung des Hinweises auf die Patenterteilung: **22.12.2010**
(21) Anmeldenummer: 07702423.0
(22) Anmeldetag: 02.02.2007
(51) Int. Cl.: C12M 1/113, C12M 1/18

(54) **VORRICHTUNG UND VERFAHREN ZUR ERZEUGUNG VON BIOGAS AUS ORGANISCHEN STOFFEN**
DEVICE AND PROCESS FOR GENERATING BIOGAS FROM ORGANIC MATERIALS
DISPOSITIF ET PROCÉDÉ DE FABRICATION DE BIOGAZ À PARTIR DE SUBSTANCES ORGANIQUES

(30) Priorität: 03.02.2006 DE 102006005066
(43) Veröffentlichungstag der Anmeldung: 15.10.2008
(73) Patentinhaber: Eltaga Licensing GmbH, 80335 München (DE)
(72) Erfinder: PERSKE, Günter, 74535 Mainhardt (DE)
(74) Vertreter: Schumacher & Willsau
(86) Internationale Anmeldenummer: PCT/DE2007/000193
(87) Internationale Veröffentlichungsnummer: WO 2007/087794

(56) Entgegenhaltungen:
- EP-A2- 0 154 334
- WO-A-82/00299
- US-A- 4 654 308
- US-A- 4 931 401

## Beschreibung

Die Erfindung betrifft eine Vorrichtung zur Erzeugung von Biogas aus organischen Stoffen, mit einem Biogasreaktor, der eine Einfüllkammer zur Befüllung mit den organischen Stoffen und einen Rücklaufkanal zum zumindest teilweisen Abführen der organischen Stoffe aus dem Biogasreaktor aufweist.

Des Weiteren betrifft die Erfindung ein Verfahren zur Biogaserzeugung.

Bei der Biogaserzeugung werden Anaerobbakterien dazu benutzt, organische Stoffe, die nicht mehr mit dem lebenden Organismus in Verbindung stehen, zu zersetzen und in Gas umzuwandeln. Anaerobe Bakterien sind das letzte Bindeglied im natürlichen Kreislauf und kommen in der Natur überall vor, z.B. in Mägen von Wiederkäuern oder im schwarzen Schlamm von Seen und Mooren. Bei der anaeroben Vergärung sind zunächst die fakultativen Methanbakterien und die obligaten Methanbakterien zu unterscheiden. Die organischen Stoffe, die bei der anaeroben Vergärung als Rohstoffe dienen, umfassen beispielsweise organische Stoffe oder Reststoffe aus Industrie, Gastronomie, Handel, Landwirtschaft (Gülle und Festmist) oder nachwachsende Rohstoffe (Maissilage, Grassilage und andere Kurzgewächse). Diese organischen Stoffe bestehen hauptsächlich aus Kohlehydraten, Fetten und Eiweißstoffen. Die fakultativen, wahlfreien Methanbakterien können auch mit Sauerstoff leben. Diese übernehmen eine erste Phase der Aufbereitung und zerlegen die organischen Stoffe in Alkohole, Fettsäuren und deren Salze.

Diese erste Phase der Aufbereitung wird als Säurebildnerphase oder Hydrolyse bezeichnet. In einer zweiten Phase übernehmen die obligaten Methanbalcterien die Umwandlung in Alkohole, Fettsäuren und deren Salze zu Gas. Diese zweite Phase bezeichnet man als Methanisierungsphase. Die erste und zweite Phase verläuft zeitversetzt um etwa sechs Stunden, wobei während der ersten sechs Stunden die so genannte Hydrolysephase abläuft.

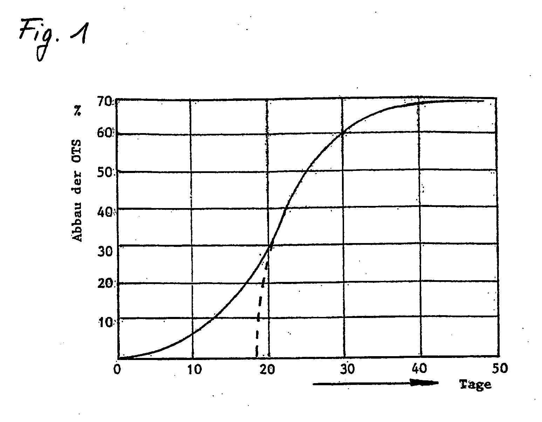

FIG. 1 zeigt ein Diagramm, welches den allgemeinen Verlauf der natürlichen Vergärung darstellt. Insbesondere zeigt das Diagramm den Abbau einer organischen Trockensubstanz (OTS) in Prozent in Abhängigkeit der verstrichenen Tage (Volllinie). Dabei ist festzustellen, dass während der ersten zwanzig Tage der Abbau der Trockensubstanz sehr langsam in Gang kommt. Nur wenige Bakterien wie in allen organischen Abfällen vorhanden, entwickeln sich bei entsprechendem Futterangebot im logarithmischen Verhältnis (gestrichelte Linie). Im gleichen Verhältnis wie sich die Bakterien entwickeln wird die organische Masse abgebaut und in Gas umgewandelt. Erstrebenswert ist ein Abbau der organischen Trockensubstanz von 70 Prozent jedoch wird dies gemäß dem Diagramm aus FIG. 1 im natürlichen Gärprozess erst nach 40 Tagen annähernd erreicht. Biogasanlagen haben zum Ziel ein Umfeld für die organische Vergärung zu schaffen, welches ermöglicht, dass diese organische Vergärung deutlich beschleunigt wird.

Eine solche Vorrichtung und ein Verfahren zur Erzeugung von Biogas ist aus der DE 30 10 183 A1 bekannt. Diese Vorrichtung umfasst die Merkmale der Oberbegriffe der Ansprüche 1 und 13. Diese Vorrichtung zur Erzeugung von Biogas leidet jedoch an einem zu geringen Prozentsatz an zu erreichendem Abbau der organischen Trockensubstanz und somit an einer zu geringen Gasausbeute.

Es ist daher die Aufgabe der vorliegenden Erfindung die gattungsgemäße Vorrichtung zur Erzeugung von Biogas und das Verfahren zur Biogaserzeugung derart weiterzubilden, dass eine höhere Gasausbeute erreicht werden kann.

Diese Aufgabe wird durch die Merkmale der unabhängigen Ansprüche gelöst.

Vorteilhafte Ausgestaltungen und Weiterbildungen der Erfindung ergeben sich aus den abhängigen Ansprüchen.

Die erfindungsgemäße Vorrichtung zur Erzeugung von Biogas baut auf dem gattungsgemäßen Stand der Technik dadurch auf, dass der Biogasreaktor des Weiteren zumindest eine Zwischenkammer aufweist, wobei die Einfüllkammer, die zumindest zwei Zwischenkammern und der Rücklaufkanal in dieser Reihenfolge Abschnitte einer nur in eine Richtung durchströmbaren Strömungsbahn für die organischen Stoffe ausbilden, wobei von zwei aufeinander folgenden Abschnitten jeweils einer eine ansteigende Strömungsbahn und der andere eine abfallende Strömungsbahn ausbildet. Durch diese Maßnahme wird erreicht, dass die Strömung ruhig und gleichmäßig in eine Richtung durch den Biogasreaktor strömt. Durch das Vorsehen zumindest einer zusätzlichen Zwischenkammer wird vermieden, dass die einzelnen Kammern zu groß werden, denn dann besteht die Gefahr, dass sich unterschiedliche Strömungszonen ausbilden und den natürlichen Ablauf der Gärung negativ beeinträchtigen. Bei unterschiedlicher Konsistenz der Gärmasse kann somit unterbunden werden, dass Strömungsspitzen der zugeführten Gärmasse bis zur Oberfläche durchstoßen, so dass nahezu unvergorene Gärmasse bzw. Frischsubstrat aus der Säurebildnerphase am Auslauf austritt. Durch die Unterteilung in mehrere Kammern werden innerhalb des Biogasreaktors mehrere Gärkammern bereitgestellt, welche die Strömung gleichmäßiger gestalten und kontrollierbar machen. Dadurch muss das frisch zugeführte Substrat die komplette Strömungsbahn zurücklegen, bevor es den Biogasreaktor wieder verlassen kann und wird somit gezwungen den kompletten Prozess in einer vorbestimmbaren Zeit zu durchlaufen. Durch diese Maßnahme kann die Ausbeute an Biogas deutlich erhöht werden.

Die erfindungsgemäße Vorrichtung zur Erzeugung von Biogas ist so weitergebildet, dass die Einfüllkammer eine Überlaufkante aufweist, mittels der die zumindest zwei Zwischenkammern von der Einfüllkammer aus mit den organischen Stoffen befüllbar ist. Durch diese Überlaufkante wird am Übergang zwischen Einfüllkammer und Zwischenkammer ein statisches Gefälle ausgebildet, an dem die Gärmasse bevorzugterweise entgasen kann. Solange die Gärmasse sich in Kammern eingeschlossen vorwärts bewegt, sind die Gase tendenziell eher eingeschlossen. Während die Gärmasse über die Überlaufkante abfällt, wird der Gärmasse die Möglichkeit gegeben, dass die Gase unter vermindertem Widerstand vollständig entweichen.

Bei der erfindungsgemäßen Vorrichtung zur Erzeugung von Biogas ist vorgesehen, dass insgesamt zumindest zwei Zwischenkammern vorgesehen sind, wobei die Zwischenkammern in einem Bereich unterhalb einer Einfüllöffnung des Rücklaufkanals und oberhalb der Überlaufkante in Verbindung stehen. Durch diese Maßnahme wird der bereits oben erwähnte Vorteil in einem noch größeren Ausmaß genutzt. Durch die Aufteilung des Gärbehälters in noch mehr Kammern ist die Strömung noch kontrollierbarer, wobei insbesondere durch die Verbindung der zwei Zwischenkammern diese nach dem Prinzip kommunizierender Gefäße miteinander in Verbindung stehen und die Gärmasse diese zwei Kammern nacheinander durchströmen muss.

Die erfindungsgemäße Vorrichtung zur Erzeugung von Biogas ist derart weitergebildet, dass die Zwischenkammern im Innenraum eines becherartigen Innenbehälters.ausgebildet sind, der im Innenraum des Biogasreaktors angeordnet ist. Dadurch ist eine konkrete Möglichkeit zur Aufteilung des Biogasreaktors in mehrere Kammern gegebein.

Des Weiteren ist die erfindungsgemäße Vorrichtung zur Erzeugung von Biogas so weitergebildet, dass von dem Rücklaufkanal ein Ablaufkanal derart abzweigt, dass der Ablaufkanal mit dem Rücklaufkanal eine kommunizierende Röhre ausbildet. Dadurch wird automatisch ein bestimmter Anteil der Gärmasse aus dem Biogasreaktor abgeführt, wenn neue Gärmasse zugeführt wird. Dabei sind keine weiteren Pumpen innerhalb des Biogasreaktors erforderlich, ebenso sind keine Ventile erforderlich, die den Ablaufkanal wahlweise öffnen und schließen.

Darüber hinaus kann die erfindungsgemäße Vorrichtung zur Erzeugung von Biogas so ausgebildet sein, dass die Querschnittsfläche der Einfüllkammer im Bereich der Überlaufkante verengt ist. Durch diese Verengung im oberen Bereich der Einfüllkammer wird das überquellende Substrat verdichtet und wirkt somit der Bildung von Schwimmschichten entgegen. Bevorzugterweise beträgt diese Querschnittsverengung 50%. Schwimmschichten würden dazu führen, dass eine Ausgasung der Gärmasse erschwert wird. Ferner wird in dieser verengten Zone die Gärmasse verdichtet und so verhindert, dass dünnflüssige Gärmasse durch zähflüssige Gärmasse hindurch oder daran vorbeifließt. Durch die Verengung erhöhen sich die inneren Reibungswiderstände im Substrat, so dass eine Endmischung nicht erfolgen kann und dadurch sicher verhindert werden kann, dass Schwimmschichten entstehen. Die Bildung von Schwimmschichten hätte zur Folge, dass der Gärprozess kippen oder sogar zum Erliegen kommen kann. Gerade im ersten Drittel des Gärablaufs entsteht das meiste Biogas und somit die größte Gefahr der Schwimmschichtenbildung.

Die selben Vorteile lassen sich dadurch erreichen, dass der Biogasreaktor einen sich nach oben verjüngenden oberen Abschnitt aufweist und die Überlaufkante sich so weit in den oberen Abschnitt erstreckt, dass.die Querschnittsfläche der Einfüllkammer im Bereich der Überlaufkante verengt wird. Zusätzlich zu den genannten Vorteilen hat dies den Effekt, dass sich durch den Übergang in den konischen Abschnitt und im konischen Abschnitt selbst die Reibung für die Gärmasse erhöht, was wie oben erläutert, der Bildung von Schwimmschichten entgegenwirkt.

Die erfindungsgemäße Vorrichtung zur Erzeugung von Biogas kann außerdem dadurch ausgebildet werden, dass der Biogasreaktor einen sich nach unten verjüngenden unteren Abschnitt aufweist, wobei im unteren Bereich des unteren Abschnittes ein Sammelraum für Sedimente ausgebildet ist, der einerseits wahlweise mit dem Innenraum des Biogasreaktors verbindbar ist und andererseits wahlweise mit der Umgebung des Biogasreaktors verbindbar ist. Durch diese Maßnahme setzen sich Sedimente bzw. Fremdstoffe wie Sand oder andere Schwerstoffe im Innenraum des Biogasreaktors nach unten ab und können dort von Zeit zu Zeit entfernt werden. Somit sind Verstopfungen der Strömungsbahnen innerhalb des Biogasreaktors ausgeschlossen. Außerdem könnten solche Sedimente den Gärprozess beeinträchtigen. Durch einen solchen entleerbaren Sammelraum ist diese Beeinträchtigung ausgeschlossen. Im vorliegenden Verfahren wird im Zyklus von etwa 10 Tagen eine ausgegorene, mehr als 90% abgebaute Masse, die kaum noch Organik enthält, mit einer Rückführrate von 50% als Impfmasse wieder in den Prozess eingeführt. Die darin befindlichen Salze und andere anorganische Teile sind nach spätestens 2 Gärzyklen über den Sammelraum auszuschleusen.

Die erfindungsgemäße Vorrichtung zur Erzeugung von Biogas kann außerdem so ausgebildet werden, dass im oberen Bereich des Biogasreaktors eine Gasabführleitung angeschlossen ist, über welche der Innenraum des Biogasreaktors mit einem konstanten Druck beaufschlagbar ist. Dadurch ist eine leicht zu realisierende, billige und sehr zuverlässige Maßnahme geschaffen, um den Druck innerhalb des Biogasreaktors konstant zu halten. Somit kann auf teure Ventile verzichtet werden, welche beim Einsatz im Milieu eines Biogasreaktors extrem schnell verschleißen.

Weiterhin kann die Vorrichtung zur Erzeugung von Biogas so ausgebildet sein, dass des Weiteren ein Flüssigkeitsbehälter vorgesehen ist, der mit einer Flüssigkeit befüllbar ist, wobei das vom Innenraum des Biogasreaktors herausführende Ende der Gasabführleitung in der Flüssigkeit platzierbar ist, so dass über die Eintauchtiefe in die Flüssigkeit, der Druck im Innenraum des Biogasreaktors einstellbar ist. Durch diesen zusätzlichen Flüssigkeitsbehälter kann der Druck innerhalb des Biogasreaktors variiert werden, wodurch sich optimale Gärbedingungen einstellen lassen. Dabei ist diese Druckeinstellung mittels Flüssigkeitsbehälter sehr einfach zu realisieren und günstig. Darüber hinaus wird das Biogas, welches über die Flüssigkeit aus der Gasabführleitung austritt, von eventuellen Schmutzpartikeln befreit.

Die erfindungsgemäße Vorrichtung zur Erzeugung von Biogas kann dadurch weitergebildet sein, dass des Weiteren eine Mischeinheit zur Befüllung der Einfüllkammer vorgesehen ist, die dazu angepasst ist, im Wesentlichen in einem 1:1 Verhältnis, neue organische Stoffe und mittels Rückführkanal aus dem Biogasreaktor abgeführte organische Stoffe zu mischen. Die aus dem Biogasreaktor abgeführten organischen Stoffe sind dabei ausgegorene und reaktionslose Stoffe. Durch eine Mischung im Verhältnis 1:1 können beste Ergebnisse erreicht werden. Da am Ende des Gärprozesses der größte Teil der organischen Stoffe abgebaut ist, liegen die Methanbakterien in höchster Konzentration vor. Die Mischung dieser aus dem Biogasreaktor abgeführten organischen Stoffe mit den neu zugeführten organischen Stoffen gewährleistet, dass der Gärprozess sehr stürmisch und schnell beginnt, denn die große Anzahl von Bakterien trifft auf hohe organische Massen und bewirkt so den stürmisch beginnenden Gärprozess.

Des Weiteren kann die Vorrichtung zur Erzeugung von Biogas dadurch weitergebildet sein, dass des Weiteren ein Wärmetauscher vorgesehen ist, der stromaufwärts der Einfüllkammer angeordnet ist und dazu angepasst ist, die neu zuzuführenden organischen Stoffe mittels eines heißen Fluids vorzuwärmen. Durch diese Vorwärmung wird erreicht, dass die Gärmasse in einer für den Gärprozess optimalen Temperatur zum Biogasreaktor zugeführt wird. Dabei steht die optimale Temperatur für eine Temperatur, bei der die Gasausbeute maximal ist.

Darüber hinaus kann die Vorrichtung für die Erzeugung von Biogas so ausgebildet sein, dass eine Zerkleinerungseinheit stromaufwärts der Einfüllkammer vorgesehen ist. Durch die Zerkleinerung der zugeführten organischen Stoffe werden die Stoffe zu einem wässrigen, pumpfähigen Gemisch einer breiten Palette organischer Bestandteile, ein ideales Futter für die Bakterien. Durch die Zerkleinerung erfolgt eine gute Durchmischung, eine bessere Pumpfähigkeit und letztlich ein sich schneller entwickelnder Gärprozess. Dadurch werden die organisches Stoffe homogenisiert, d.h. aufgeschlissen, so dass die Struktur der organischen Teile schonend zerschlagen wird und das eingeschlossene Wasser freigesetzt wird. Dadurch wird eine große Oberfläche geschaffen und die Bakterien können sich intensiver ansiedeln.

Als Weiterentwicklung kann die Vorrichtung erfindungsgemäß so ausgebildet sein, dass ein Gasspeicher vorgesehen ist, in dem im Biogasreaktor erzeugtes Biogas speicherbar ist und der den Biogasreaktor zumindest teilweise umgibt. Diese Weiterbildung hat den Vorteil, dass der Gasbehälter eine Isolationswirkung gegenüber dem Biogasreaktor entfaltet, so dass die Wärme besser im Biogasreaktor verbleibt und dem Biogasreaktor weniger Wärmeenergie zugeführt werden muss. Gleichzeitig stellt diese Weiterentwicklung ohne zusätzliche Bauteile einen Behälter zum Speichern von Biogas zur Verfügung.

Der Wärmetauscher für eine Biogasanlage baut auf dem gattungsgemäßen Stand der Technik durch eine Reinigungseinrichtung auf, die bei geschlossenem Wärmetauschergehäuse über die Wärmeeintragungskörper streifbar ist. Die Problematik bei einem Wärmetauscher im Zusammenhang mit dickflüssiger organischer Masse besteht darin, dass ab etwa 60°C Eiweiß ausflockt und sich an den Wärmeeintragungskörpern absetzt. Der Wärmetauscher ermöglicht, dass sich solche Anhaftungen ständig entfernen lassen um einen guten Durchfluss und eine gute Wärmeübertragung zu gewährleisten. Außerdem wird durch eine bewegbare Reinigungseinrichtung eine gute Durchmischung der Masse erreicht, wodurch diese gleichmäßig erwärmt wird.

Des Weiteren kann der Wärmetauscher derart weitergebildet sein, dass die Reinigungseinrichtung mittels eines Spindelantriebs hin und her bewegbar ist. Mittels des Spindelantriebs lässt sich eine Bewegung der Reinigungseinrichtung wartungsarm und kostengünstig realisieren.

Darüber hinaus kann der Wärmetauscher so ausgebildet sein, dass die Wärmeeintragungskörper doppelwandige Rohre sind, in denen der Vorlauf den Rücklauf ummantelt oder der Rücklauf den Vorlauf ummantelt. Durch die Verwendung doppelwandiger Rohre kann der Vorlauf und Rücklauf des heißen Fluids auf der gleichen Seite aus dem Gehäuse des Wärmetauschers austreten. Dadurch lässt sich der Wärmetauscher auf der gegenüberliegenden Wärmetauscherseite einfacher öffnen. Darüber hinaus legt das heiße Fluid durch die doppelwandigen Rohre innerhalb des Wärmetauschers die doppelte Strecke zurück als wenn man normale einwandige Rohre vorsehen würde.

Mit dem erfindungsgemäßen Verfahren zur Biogaserzeugung werden die oben beschriebenen Vorteile in übertragener Weise erreicht.

In vorteilhafter Weise kann das Verfahren so weitergebildet sein, dass die innerhalb des Biogasreaktors ablaufenden Verfahrensschritte ohne aktives Umrühren der organischen Stoffe ausgeführt werden. Durch die Vermeidung von Umrühren der organischen Stoffe wird eine Störung des Gärprozesses vermieden. Die Hydrolysephase ist sensibel und verträgt keine Störungen.

Darüber hinaus kann das Verfahren derart weitergebildet sein, dass die neu zugeführten organischen Stoffe vor dem Einfüllen in die Einfüllkammer mittels eines Wärmetauschers auf 35°C - 37°C vorgewärmt werden. Dadurch läuft der Gärprozess im mesophilen Bereich ab, wodurch höhere Abbauraten und somit eine größere Gasmenge erreicht werden kann. Verfahren vom Stand der Technik arbeiten häufig im thermophilen Bereich (ca. 55°C), wodurch eine Hygienisierung und ein schneller Prozessablauf erreicht wird, mehr Gas wird dadurch jedoch nicht erzeugt. Im mesophilen Bereich ist die Energiebilanz besser, weil weniger Energie zum Aufheizen benötigt wird.

Außerdem kann das Verfahren derart weitergebildet sein, dass die in die Einfüllkammer eingefüllten organischen Stoffe aus neu zugeführten vorgewärmten organischen Stoffen und direkt mittels Rücklaufkanal abgeführten organischen Stoffen vermischt werden. Durch diese Vermischung mit organischen Stoffen, die bereits im Biogasreaktor waren, werden die neu zugeführten organischen Stoffe mit voll aktiven Bakterien durchmischt, so dass der Gärprozess sehr schnell beginnen kann.

Des Weiteren kann beim Verfahren vorgesehen sein, dass das Mischungsverhältnis 1:1 beträgt. Dieses Mischungsverhältnis hat sich als optimales Mischungsverhältnis für maximale Gasausbeute erwiesen.

Schließlich kann das erfindungsgemäße Verfahren so weitergebildet sein, dass im Innenraum des Biogasreaktors ein Innendruck konstant gehalten wird. Durch den konstanten Innendruck wird der Gärprozess möglichst wenig gestört was sich vorteilhaft auf den Gärprozess auswirkt. Durch den konstanten Innendruck werden unkontrollierte Strömungen der Gärmasse verhindert.

Eine bevorzugte Ausführungsform der Erfindung wird nachfolgend anhand der Figuren beispielhaft erläutert.

Es zeigen:
- FIG. 1: ein Diagramm, welches den allgemeinen Verlauf der natürlichen Vergärung darstellt;
- FIG. 2: eine schematische Darstellung der erfindungsgemä- ßen Vorrichtung zur Erzeugung von Biogas;
- FIG. 3: eine Draufsicht einer Zerkleinerungseinheit der Vorrichtung aus FIG. 2;
- FIG. 4: einen Verlauf der Vergärung, den das Verfahren der vorliegenden Erfindung sicher erreicht;
- FIG. 5: eine schematische Schnittdarstellung eines Wärme- tauschers der Vorrichtung aus FIG. 2;
- FIG. 6: eine schematische Schnittdarstellung entlang der Linie I-I aus FIG. 5;
- FIG. 7: eine schematische Schnittdarstellung entlang der Linie II-II aus FIG. 5; und
- Fig. 8: eine schematische Darstellung einer Weiterbildung der erfindungsgemäßen Vorrichtung zur Erzeugung von Biogas.

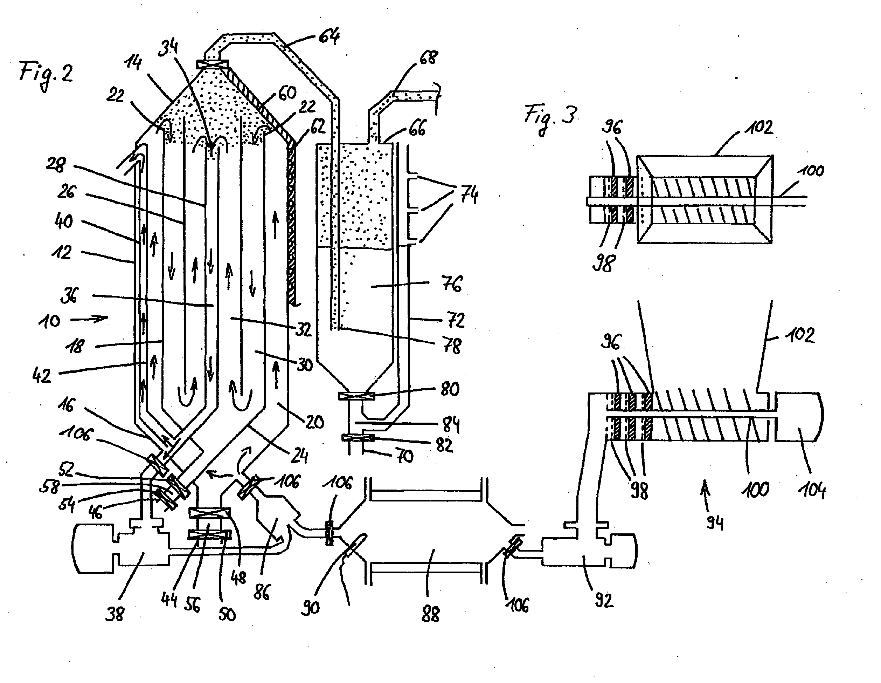

FIG. 2 zeigt eine schematische Darstellung der erfindungsgemäßen Vorrichtung zur Erzeugung von Biogas. In der bevorzugten Ausführungsform umfasst der Biogasreaktor 10 einen Außenbehälter 12, der vorzugsweise in einem mittleren Abschnitt zylindrisch und in einem oberen Abschnitt 14 sowie in einem unteren Abschnitt 16 jeweils zum Ende hin konisch verjüngt ist. Im Inneren des Außenbehälters 12 ist ein Innenbehälter 18 aufgenommen, der becherförmig ist und im Wesentlichen mit konstantem Abstand zum Außenbehälter 12 angeordnet ist, so dass zwischen dem Außenbehälter 12 und dem Innenbehälter 18 eine Einfüllkammer 20 ausgebildet wird, die den Innenbehälter 18 einhüllt. Der Außenbehälter 12 sowie der Innenbehälter 18 sind vorzugsweise aus Stahl, jedoch ist auch eine Ausführung mit anderen Werkstoffen wie beispielsweise Kunststoffen realisierbar, Eine Oberkante des Innenbehälters 18, die im vorliegenden Ausführungsbeispiel als Überlaufkante 22 fungiert, ist soweit in den sich nach oben verjüngenden oberen Abschnitt 14 geführt, dass sich die Querschnittsfläche der dazwischen liegenden Einfüllkammer 20 nach oben hin um etwa 50% verengt. Jedoch ist in diesem Zusammenhang lediglich wesentlich, dass sich der Querschnitt am Ende der Einfüllkammer 20 verengt. Alternativ kann dies auch dadurch erreicht werden, dass die obere Spitze der Überlaufkante 22 nach Außen, also hin zum Außengehäuse 12, gebogen ist. Ebenso ist möglich, dass die Querschnittsverengung nicht durch Zusammenwirken des sich nach oben verjüngenden oberen Abschnitts 14 mit der Überlaufkante 22 ergibt, sondern ein separates Leitblech am oberen Ende der Einfüllkammer 20 vorgesehen ist, welches am Außengehäuse 12 befestigt ist und den Querschnitt der Einfüllkammer 20 nach oben hin verengt, so dass sich die benötigte Querschnittsverengung ergibt. Im unteren Bereich ist der Innenbehälter 18 ähnlich zum Außenbehälter 12 nach unten verjüngt. Der Innenbehälter 18 ist vorzugsweise zylinderförmig und im sich verjüngenden unteren Abschnitt 24 konisch ausgebildet. Innerhalb des Innenbehälters 18 ist eine zylinderförmige Innenröhre 26 so angeordnet, dass zwischen Innenröhre 26 und Innenbehälter 18 im Wesentlichen der gleiche Abstand ausgebildet wird, wie zwischen-Innenbehälter 18 und Außenbehälter 12. Die Unterkante der Innenröhre 26 erstreckt sich fast so weit nach unten wie der zylindrische Abschnitt (der nicht verjüngte Abschnitt) des Innenbehälters 18. Die Oberkante der Innenröhre 26 erstreckt sich weiter nach oben als die Überlaufkante 22. Im Inneren der Innenröhre 26 befindet sich ein Rücklaufrohr 28, welches sich im Inneren der Innenröhre 26 nach unten in den Abschnitt 24 des Innenbehälters 18 erstreckt, wo das Rücklaüfrohr 28 aus dem Innenbehälter 18 austritt. Das Rücklaufrohr 28 erstreckt sich so weit nach oben, dass die Oberkante des Rücklaufrohrs 28 hinsichtlich der Vertikalen unterhalb der Überlaufkante 22 platziert ist. Vorteilhafterweise erstreckt sich die Oberkante des Rücklaufrohrs 28 im Wesentlichen so weit nach oben wie der mittlere (vorzugsweise zylindrische) Abschnitt des Außenbehälters 12. Im vorliegenden Ausführungsbeispiel sind der Außenbehälter 12, der Innenbehälter 18, die Innenröhre 26 und das Rücklaufrohr 28 konzentrisch angeordnet. Zwischen der Außenseite der Innenröhre 26 und der Innenseite des Innenbehälters 18 wird eine erste, im Wesentlichen zylinderförmige Zwischenkammer 30 ausgebildet. Zwischen der Außenseite des Rückläufrohres 28 und der Innenseite der Innenröhre 26 wird eine zweite, im Wesentlichen zylinderförmige Zwischenkammer 32 ausgebildet. Die erste Zwischenkammer 30 und die zweite Zwischenkammer 32 stehen im unteren Bereich miteinander in Verbindung. Die Oberkante des Rücklaufrohres 28 bildet eine Einfüllöffnung 34 aus. Im Inneren des Rücklaufrohres 28 ist ein Rücklaufkanal 36 ausgebildet. Das Rücklaufrohr 28 führt wie bereits erwähnt im unteren Abschnitt 24 des Innenbehälters 18 aus dem Innenbehälter 18 heraus, tritt im unteren Abschnitt 16 durch die Wandung des Außenbehälters 12 und führt in eine Impfpumpe 38, die vorzugsweise eine Exenterschneckenpumpe ist. Am Abschnitt des Rücklaufrohres 28, welcher innerhalb der Einfüllkammer 20 verläuft, zweigt ein Ablaufrohr 40 ab, welches sich in der Einfüllkammer 20 so weit nach oben erstreckt, dass sich eine obere Öffnung des Ablaufrohres 40 in etwa auf gleicher Höhe wie die Einfüllöffnung 34 des Rücklaufrohres 28 befindet. Das Ablaufrohr 40 ist im oberen Bereich so ausgeführt, dass der obere Abschnitt des Rohres um mehr als 90 Grad umgebogen ist und sich der umgebogene Abschnitt durch die Wandung des Außenbehälters 12 nach Außen erstreckt. Der von dem Ablaufrohr 40 ausgebildete Ablaufkanal 42 ist somit U-förmig mit dem Rücklaufkanal 36 verbunden, so dass der Rücklaufkanal 36 und der Ablaufkanal 42 eine kommunizierende Röhre ausbilden. Die Einfüllkammer 20 ist so ausgeführt, dass sie in einem unteren Bereich von Außen mit organischen Stoffen bzw. einer organischen Substanz befüllbar ist. Die organische Substanz wird auf eine später genauer erläuterte Art und Weise auch durch die Einfüllkammer 20, die erste Zwischenkammer 30 und die zweite Zwischenkammer 32 gefördert, wobei die organische Substanz noch Sedimente oder Schwerstoffe aufweisen kann. Daher zweigt am untersten Ende des Außenbehälters 12 und des Innenbehälters 18 jeweils ein Rohrstück 44 und 46 ab, welches nahe des jeweiligen Behälters mit einem Schieber 48, 52 versehen ist und in einem gewissen Abstand dazu mit einem weiteren Schieber 50, 54 versehen ist. Mit den jeweiligen Schiebern kann das jeweilige Rohrstück 44, 46 wahlweise geöffnet und geschlossen werden. Der Abstand des Schiebers 50 vom Schieber 48 beträgt vorzugsweise etwa 80 cm und der Abstand des Schiebers 52 zum Schieber 54 beträgt vorzugsweise 60 cm. Im Normalbetrieb sind die Schieber 48 und 52 geöffnet und die Schieber 50 und 54 geschlossen. Wenn sich somit im organischen Substrat befindliche Sedimente nach unten absetzen, gleiten diese entlang der Abschnitte 16 und 24 zur Mitte des jeweiligen Behälters 12, 18 und verlassen diesen durch den jeweiligen geöffneten Schieber 48, 52 in das jeweilige Rohrstück 44, 46. Dort werden die Sedimente an den geschlossenen Schiebern 50, 54 angesammelt. Der Rohrabschnitt zwischen dem Schieber 48 und 50 sowie der Rohrabschnitt zwischen dem Schieber 52 und 54 bilden demnach jeweils einen Sammelraum 56, 58 für Sedimente aus. Vorzugsweise sind die Rohrabschnitte bei den Sammelräumen 56, 58 durchsichtig ausgebildet, beispielsweise mittels Plexiglas, so dass die Menge an angesammelten Sedimenten überwacht werden kann.
Bei Erreichen einer bestimmten Menge können die angesammelten Sedimente entleert werden, indem die jeweiligen Schieber 48 und 52 geschlossen werden, um ein Auslaufen der Behälter 12, 18 zu verhindern. Dann werden die jeweiligen Schieber 50 und 54 geöffnet und die Sammelräume 56, 58 entleert. Für den Normalbetrieb werden die Schieber 50 und 54 wieder geschlossen und die Schieber 48 und 52 geöffnet: Eine nur abschnittsweise dargestellte Isolation 60 umgibt den Außenbehälter 12 vollständig (die Zu- und Ableitungen sind ausgespart), so dass die für die Erzeugung von Biogas vorteilhafte Temperatur von vorzugsweise 35°C im Inneren des Biogasreaktors 10 möglichst konstant gehalten werden kann und damit weniger Energie zum Aufrechterhalten dieser Temperatur zugeführt werden muss. In die Isolation 60 ist eine Heizung 62 eingebettet, die im bevorzugten Ausführungsbeispiel in Form von spiralförmig angeordneten Wasserleitungen ausgebildet ist, welche Wasser führen, das beispielsweise in einem nicht dargestellten Blockheizkraftwerk erwärmt wird. Alternativ können ebenso Heizdrähte in die Isolation 60 eingebettet sein. Vorzugsweise umgibt die Heizung 62 den Außenbehälter 12 von unten bis unterhalb des oberen Abschnittes 14. Zum Schutz der Isolation 60 kann die Isolation 60 samt Heizung 62 von einem Schutzmantel, wie beispielsweise einem Blechmantel, umgeben sein.

Am oberen Ende des Außenbehälters 12, dies ist das verjüngte Ende des oberen Abschnittes 14 ist eine Gasabführleitung 64 abgezweigt. Diese Gasabführleitung 64 ist außerhalb des Außenbehälters 12 neben diesem nach unten geführt, wobei ein Endabschnitt der Gasabführleitung 64 in einen Flüssigkeitsbehälter 66 eintritt und sich innerhalb dieses Flüssigkeitsbehälters 66 nach unten erstreckt. Der Flüssigkeitsbehälter 66 ist vorzugsweise ein zylindrischer Behälter dessen unterer Abschnitt sich konisch nach unten verjüngt. An der Oberseite des Flüssigkeitsbehälters 66 ist eine Gaseinspeisleitung 68 abgezweigt, über die das gewonnene Biogas einem nicht dargestellten Gasspeicher zugeführt wird, von dem aus es einem nicht dargestellten Blockheizkraftwerk zur Verstromung zur Verfügung steht. Am unteren Ende des Flüssigkeitsbehälters 66 ist ein Rohrstück 70 aus dem Flüssigkeitsbehälter 66 herausgeführt. Von diesem Rohrstück 70 zweigt ein Steigrohr 72 ab, das neben dem Flüssigkeitsbehälter 66 bis zur Oberkante des Flüssigkeitsbehälters 66 nach oben geführt ist. Das Steigrohr 72 ist oben offen und zwischen der Oberkante des Steigrohres 72 und mehr als 1 m unterhalb der Oberkante sind drei Öffnungen 74 ausgebildet, wobei sich die Unterste der Öffnungen mehr als 1 m unterhalb der Oberkante des Steigrohres 72 befindet. Der Abstand zwischen der Untersten der drei Öffnungen 74 und der Obersten der drei Öffnungen 74 beträgt vorzugsweise 1m. Das Steigrohr 72 ist nach dem Prinzip kommunizierender Röhren mit dem Innenraum des Flüssigkeitsbehälters 66 verbunden. Der Innenraum des Flüssigkeitsbehälters 66 ist im Betrieb mit einer Flüssigkeit 76, vorzugsweise Wasser, befüllt deren Flüssigkeitspegel sich mittels der Öffnungen 74 einstellen lässt. Durch das Prinzip der kommunizierenden Röhren herrscht im Steigrohr 72 der gleiche Flüssigkeitspegel wie im Flüssigkeitsbehälter 66, so dass falls die Unterste der Öffnungen 74 geöffnet ist, der Flüssigkeitsbehälter 66 bis zu einem Pegel mit Flüssigkeit 76 befüllt werden kann, der dem Pegel der Untersten der Öffnungen 74 entspricht. Wird die Unterste der Öffnungen 74 verschlossen, beispielsweise mittels eines Pfropfens, so ist der Flüssigkeitsbehälter 66 mit einem höheren Flüssigkeitspegel befüllbar, der einem Pegel der weiter oben befindlichen Öffnungen 74 entspricht. Falls alle Öffnungen 74 verschlossen sind, kann der Flüssigkeitsbehälter 66 vollständig befüllt werden, wobei bei Erreichen der vollständigen Befüllung die Flüssigkeit bis zur Oberkante des Steigrohres 72 reicht. Das aus dem Außenbehälter 12 herausgeführte Ende 78 der Gasabführleitung 64 ist so innerhalb des Flüssigkeitsbehälters 66 angeordnet, dass dieses Ende 78 in die Flüssigkeit 76 eingetaucht ist. Die untere Öffnung des Endes 78 ist 2 m von der Obersten der Öffnungen 74 des Steigrohres 72 beabstandet. Die Eintauchtiefe der Gasabführleitung 64 in die Flüssigkeit 76 beträgt somit minimal 1 m, wenn die Unterste der drei Öffnungen 74 geöffnet ist, und maximal 2 m, wenn nur die Oberste der drei Öffnungen 74 geöffnet ist. Durch diese einstellbare Eintauchtiefe des Endes 78 der Gasabführleitung 64 kann der Druck innerhalb des Außenbehälters 12 auf einen konstanten Druck eingestellt werden. Bei Befüllung mit Wasser wird somit mit einer Eintauchtiefe von 1 m ein Druck von 0,1 bar im Außenbehälter 12 erreicht. Bei einer Eintauchtiefe von 2 m wird ein Druck von 0,2 bar im Außenbehälter 12 eingestellt. Am unteren Ende des Flüssigkeitsbehälters 66 ist wie vorstehend beschrieben das Rohrstück 70 herausgeführt. Dabei ist im Rohrabschnitt zwischen dem Austritt am Flüssigkeitsbehälter 66 und der Abzweigung des Steigrohrs 72 ein Schieber 80 sowie im Rohrabschnitt nach der Abzweigung des Steigrohres 72 ein Schieber 82 vorgesehen. Mit diesen beiden Schiebern 80, 82 kann der Durchfluss durch das Rohrstück 70 wahlweise geöffnet oder geschlossen werden. Im Normalbetrieb ist der Schieber 80 geöffnet und der Schieber 82 geschlossen, wodurch ein Sammelraum 84 für Sedimente ausgebildet wird. Somit werden im Biogas enthaltene Verunreinigungen durch die Flüssigkeit 76 ausgefiltert. Das Gas steigt in der Flüssigkeit 76 nach oben und die herausgefilterten Sedimente setzen sich in der Flüssigkeit 76 nach unten ab, werden dort durch die sich verjüngende Form des unteren Abschnittes des Flüssigkeitsbehälters 66 zur Mitte geführt und sammeln sich im Sammelraum 84. Im Bereich dieses Sammelraums 84 kann das Rohrstück 70 durchsichtig, beispielsweise mittels Plexiglas ausgeführt sein, so dass die Ansammlung an Sedimenten überwacht werden kann. Wenn die Ansammlung an Sedimenten im Sammelraum 84 eine bestimmte Menge erreicht hat, kann der Schieber 80 geschlossen werden und der Schieber 82 geöffnet werden, so dass am unteren Ende des Rohrstücks 70 die Sedimente aus dem System entleert werden können. Nach dem Entleeren des Sammelraums 84 wird der Schieber 82 geschlossen und der Schieber 80 wieder geöffnet.

Wie vorstehend erwähnt, kann die Einfüllkammer 20 von unten befüllt werden. Dazu erstreckt sich durch die Wandung des Außenbehälters 12 im unteren Abschnitt 16 ein Rohrstück, das die Einfüllkammer 20 mit dem Ausgang einer Mischeinheit 86 verbindet. Der Ausgang der Mischeinheit 86 verjüngt sich zur Einfüllkammer 20 hin, vorzugsweise um 50%. Die Eingänge der Mischeinheit 86 sind mit Rohren verbunden, mittels derer die Mischeinheit 86 mit den Ausgängen der Impfpumpe 38 und eines Wärmetauschers 88 verbunden sind. Die Mischeinheit 86 mischt organische Stoffe, die von der Impfpumpe 38 und vom Wärmetauscher 88 zugeführt werden vorzugsweise in einem 1:1 Verhältnis. Alternativ kann die Vorrichtung zur Biogaserzeugung auch mit anderen Mischungsverhältnissen betrieben werden. Der später genauer erläuterte Wärmetauscher 88 weist einen Temperatursensor 90 auf, der nahe des Ausganges angeordnet ist und mit dem die Temperatur des im Wärmetauscher befindlichen organischen Substrats ermittelt werden kann. Der Wärmetauscher 88 ist eingangsseitig mit einer Frischsubstrat-Pumpe 92 verbunden, welche vorzugsweise eine Exenterschneckenpumpe ist. Diese Frischsubstrat-Pumpe 92 wiederum ist eingangsseitig mit einer Zerkleinerungseinheit 94 verbunden. Die Verbindung zwischen Zerkleinerungseinheit 94 und Frischsubstrat-Pumpe 92 sowie die Verbindung zwischen Frischsubstrat-Pumpe 92 und Wärmetauscher 88 wird mittels Rohrverbindungen realisiert. Die Zerkleinerungseinheit 94 umfasst drei hintereinander angeordnete Schneidewerkzeuge 96, wie beispielsweise sternförmige Schneidemesser, an deren Substratstrom-abgewandten Seiten jeweils eine Lochscheibe 98 angeordnet ist. Die Schneidewerkzeuge 96 sind auf einer Welle 100 angeordnet. Diese Welle 100 ist zumindest abschnittsweise als eine Förderschneckenwelle ausgebildet. Zur Verbindung der Welle 100 mit den Schneidewerkzeugen 96 ist die Welle 100 im Bereich der Schneidewerkzeuge 96 angeflacht, so dass die Schneidewerkzeuge 96 passgenau aufgesetzt werden können. Die Lochscheiben 98 weisen ein mittiges Rundloch auf, durch welches die Welle 100 hindurchgeführt ist, so dass die Lochscheiben 98 die Drehung der Welle 100 nicht beeinträchtigen. Die Lochscheiben 98 werden durch einen nicht dargestellten Bolzen arretiert, so dass an der jeweiligen Lochscheibe 98 das Schneidewerkzeug 96 eine schneidende und scherende Wirkung auf das zugeführte organische Frischsubstrat ausübt, welches fasrig sein kann. Die Lochgröße der Lochscheiben 98 ist abgestuft, so dass die Zerkleinerung stufenweise vorgenommen wird und individuell angepasst werden kann. Somit wird über einen Einfülltrichter 102 zugeführtes organisches Frischsubstrat mittels der als Förderschnecke ausgebildeten Welle 100 zu den Schneidewerkzeugen 96 zugeführt, von diesen zerkleinert und zur Frischsubstrat-Pumpe 92 weitergefördert. Dabei werden die Welle 100 und somit die Schneidewerkzeuge 96 von einer Antriebseinheit 104 (beispielsweise ein Elektromotor) angetrieben. Eine Draufsicht der Zerkleinerungseinheit ist in FIG. 3 dargestellt.

Zwischen Frischsubstrat-Pumpe 92 und Wärmetauscher 88, zwischen Wärmetauscher 88 und Mischeinheit 86, zwischen Mischeinheit 86 und Außenbehälter 12, zwischen Impfpumpe 38 und Außenbehälter 12 sowie zwischen Außenbehälter 12 und Flüssigkeitsbehälter 66 ist jeweils ein Schieber 106 angeordnet, mit dem die jeweiligen Rohrverbindungen wahlweise geöffnet und geschlossen werden können. Im Normalbetrieb sind all diese Schieber 106 geöffnet, jedoch kann es beispielsweise wartungsbedingt erforderlich sein, dass beim Austausch einer Komponente die jeweiligen der Komponente vor- und/oder nachgeschalteten Schieber 106 geschlossen werden, um einen Austausch der Komponente zu ermöglichen, ohne dass organische Stoffe aus dem System austreten.

Nachfolgend wird der Betrieb der Vorrichtung zur Erzeugung von Biogas aus FIG. 2 beziehungsweise ein Verfahren zur Erzeugung von Biogas unter Verwendung der Vorrichtung aus FIG. 2 beschrieben. Bei Verfahren vom Stand der Technik wurde zu wenig beachtet, dass die beiden Arten an Methanbakterien der fakultativen Methanbakterien und die.obligaten Methanbakterien in Symbiose leben, d.h. sie ergänzen sich und sind voneinander abhängig. Die eingangs beschriebene erste Phase (Hydrolyse oder Säurebildnerphase) und zweite Phase (Methanisierungsphase) des Gärprozesses verlaufen zeitversetzt um etwa sechs Stunden, wobei während der ersten sechs Stunden die so genannte Hydrolysephase abläuft. Die vorbereiteten Alkohole und Fettsäuren müssen auch in der danach ablaufenden zweiten Phase verarbeitet werden können. Dazu ist es wesentlich, dass keine Störungen durch Rühren oder Mischen wie im erfindungsgemäßen Verfahren durch eine erneute, überhöhte Säurebildung die erste Phase aus dem Gleichgewicht bringen. Jede Zugabe von Frischsubstrat aktiviert die Säurebildung, so dass es zur Anhäufung von Säureprodukten kommt, wenn die Aufbereitung der organischen Stoffe nicht optimal funktioniert, erliegt der eigentliche Gärprozess der Überproduktion von Säureprodukten. Daher wurde im vorliegenden Verfahren bewusst die Säurebildnerphase (Hydrolyse) in den Vordergrund gerückt, wodurch sich das vorliegende Verfahren von vielen Verfahren aus dem Stand der Technik unterscheidet. Im vorliegenden Verfahren sind Hydrolyse und Methanisierung im Gleichgewicht und werden nicht wie bei Verfahren aus dem Stand der Technik getrennt durchgeführt. Würde man die Hydrolyse und Methanisierung getrennt voneinander durchführen, müsste man ein vorgesäuertes Substrat in den aktiven Prozess einschleusen, was zur Folge hätte, dass sich Säurekonzentrationen bilden und der Prozess lange Zeit für die Gleichgewichtsherstellung benötigt, teilweise 20 bis 30 Tage und mehr. Der gleiche Nachteil entsteht durch Rühren und Mischen sowie Einblasen von Gas. Im vorliegenden Prozess wird bewusst auf Störungen dieses Gleichgewichtes durch Rühren und Mischen verzichtet.

Vor dem Einfüllen der organischen Stoffe in den Einfülltrichter 102 der Zerkleinerungseinheit 94 ist es vorteilhaft, die organischen Stoffe eventuell auszusortieren, d.h. grobe Störstoffe entfernen und größere Teile auf ca. 30 mm zu zerkleinern. Diese organischen Stoffe werden dann in den Einfülltrichter 102 eingefüllt. Dann wird die Antriebseinheit 104 manuell oder automatisch (z.B. mittels nicht dargestellter Lichtschranke usw.) eingeschaltet. Dadurch dreht sich die Welle 100, so dass die Förderschnecke der Welle 100 das eingefüllte Substrat zum Ersten der drei Schneidewerkzeuge 96 fördert. Dieses Schneidewerkzeug 96 zerkleinert das organische Frischsubstrat und fördert dies durch die entsprechende Lochscheibe 98. Anschließend durchläuft das organische Frischsubstrat die zweite und dritte Zerkleinerungsstufe (von rechts nach links in FIG. 2) bestehend aus Schneidewerkzeug 96 und zugehöriger Lochscheibe 98. Durch diese Zerkleinerung werden die organischen Stoffe aus Gastronomie, aus Fettabscheidern, aus der Lebensmittelindustrie und anderen Quellen homogenisiert, d.h. so aufgeschlissen, dass die Struktur der organischen Teile schonend zerschlagen wird und das eingeschlossene Wasser freigesetzt wird. Dadurch wird eine große Oberfläche geschaffen und die in den Gärprozess involvierten Bakterien können sich intensiver ansiedeln. Das somit durch die Schneidewerkzeuge 96 gewonnene Substrat ist ein wässriges, pumpfähiges Gemisch einer breiten Palette organischer Bestandteile. Die Förderwirkung der Welle 100 und die Förderwirkung der Schneidewerkzeuge 96 transportiert das organische Frischsubstrat weiter zur Frischsubstrat-Pumpe 92. Diese Frischsubstrat-Pumpe 92 wird mittels einer nicht dargestellten Steuerung angesteuert und pumpt das Frischsubstrat in den Wärmetauscher 88 und von dort weiter in die Mischeinheit 86 und schließlich in die Einfüllkammer 20 des Biogasreaktors 10. Dabei wird das von der Zerkleinerungseinheit 94 zugeführte Frischsubstrat zunächst in den Innenraum des Wärmetauschers 88 gefördert. Dann schaltet die Frischsubstrat-Pumpe 92 ab. Das in den Wärmetauscher 88 zugeführte Frischsubstrat wird erwärmt, im vorliegenden Ausführungsbeispiel auf 37°C, was mittels des Temperatursensors 90 überwacht wird. Diese Erwärmung wird erreicht, indem in Wärmeeinträgungskörper, welche später erläutert werden, heißes Fluid eingeleitet wird, welches räumlich vom Frischsubstrat getrennt ist. Dieses Fluid hat vorzugsweise eine Temperatur von etwa 80°C. Sobald der Temperatursensor 90 erfasst, dass die Temperatur von 37°C erreicht ist, wird die Frischsubstrat-Pumpe 92 eingeschaltet, so dass neues Frischsubstrat in den Wärmetauscher 88 eingetragen wird und das vorgewärmte Frischsubstrat aus dem Wärmetauscher 88 austritt und in die Mischeinheit 86 eintritt. Mit der Frischsubstrat-Pumpe 92 wird gleichzeitig auch die Impfpumpe 38 betrieben, was später genauer erläutert wird. Die Frischsubstrat-Pumpe 92 bleibt vorzugsweise so lange eingeschaltet, bis der Temperatursensor 90 eine Temperatur gleich oder kleiner 35°C erfasst. Dann wird die Frischsubstrat-Pumpe 92 ausgeschaltet, so dass das neu in den Wärmetauscher 88 zugeführte organische Frischsubstrat, welches noch nicht vorgewärmt ist, nun vorgewärmt werden kann bis es eine Temperatur von 37°C erreicht und wie vorstehend beschrieben weitertransportiert wird. Die Intervalle, in denen das organische Frischsubstrat zugeführt wird, können variabel gestaltet werden und die Frischsubstrat-Pumpe 92 muss nicht ausschließlich abhängig vom Temperatursensor 90 gesteuert werden. Vielmehr ist die Steuerung mittels Temperatursensor 90 so zu verstehen, dass eine Grundvoraussetzung für die Zufuhr des Frischsubstrates ist, dass dieses eine minimale Temperatur von 35°C aufweist. Die Intervalle können auch länger sein, wie für die Vorwärmung des Frischsubstrats im Wärmetauscher 88 erforderlich. Somit kann bei schneller Vorwärmung des Frischsubstrates im Wärmetauscher 88 eine nahezu kontinuierliche Zufuhr oder eine Zufuhr von Frischsubstrat in bestimmten Zyklen erfolgen. Die Impfpumpe 38 dient dazu, aus dem Biogasreaktor 10 abgeführtes Substrat, welches ebenfalls eine Temperatur von 35°C aufweist, in die Mischeinheit 86 einzuspeisen. Wie vorstehend erwähnt, werden die Frischsubstrat-Pumpe 92 und die Impfpumpe 38 synchron betrieben, so dass jeweils gleiche Anteile an Substrat von der Impfpumpe 38 und der Frischsubstrat-Pumpe 92 in die Mischeinheit 86 eingespeist werden. Es hat sich gezeigt, dass die Zusammenmischung von aus dem Biogasreaktor austretendem Substrat (reaktionslose Gärmasse) mit Frischsubstrat (aus dem Wärmetauscher 88 abgeführtes Substrat) im Verhältnis 1:1 die besten Ergebnisse liefert, vorausgesetzt, dass beide Masseströme annähernd die gleichen Temperaturen haben. Eine Töleranz von 1 - 2°C ist jedoch hinnehmbar. Durch die Zusammenmischung von reaktionsloser Gärmasse mit Frischsubstrat im richtigen Verhältnis tritt eine Belebung im Gärprozess ein, so dass schließlich die restlichen organischen Stoffe angegriffen und abgebaut werden. Diese Maßnahme trägt wesentlich zur hohen Abbaurate der organischen Stoffe des vorliegenden Verfahrens bei, welche über 70% und höher liegen kann. Dabei ist darauf zu achten, dass diese Vermischung der beiden Massenströme auf keinen Fall in einem vorgeschalteten Behälter, wie zum Beispiel einer Vorgrube, erfolgen darf, sonst geht Gas verloren.

In der Mischeinheit 86 wird das zugeführte Substrat durch den konisch verjüngten Ausgang der Mischeinheit 86 weiter vermischt. Am Ende eines Gärprozesses ist der größte Teil der organischen Stoffe abgebaut und die Methanbakterien liegen in höchster Konzentration vor. Durch diese Einimpfung der Methanbakterien in das Frischsubstrat mittels der Mischeinheit 86 ist gewährleistet, dass der Gärprozess sehr stürmisch beginnt, denn die große Anzahl von Bakterien trifft auf eine große Masse an Frischsubstrat und bewirkt so den stürmisch beginnenden Gärprozess. Nach Eintritt in die Einfüllkammer 20 steigt das Substrat in der Einfüllkammer 20, getrieben von nachfolgendem Substrat, nach oben.

Das in der Einfüllkammer 20 nach oben strömende Substrat wird bei Erreichen der Verengung im oberen Bereich der Einfüllkammer 20 verdichtet, so dass keine Schwimmschichten entstehen. Die an der Überlaufkante 22 überquellende Gärmasse stürzt über die Überlaufkante 22 in die erste Zwischenkammer 30. Bei diesem Stürzen über die Überlaufkante 22 entgast die Gärmasse vollständig nach oben. Zudem werden eventuelle Zusammenballungen des Substrats aufgebrochen, die das Entgasen ermöglichen und unterstützen, Die Abstürzhöhe an dieser Überlaufkante 22 beträgt in der Praxis ca. 0,6 m und hängt mitunter vom Druck ab, mit dem der Innenraum des Biogasreaktors 10 beaufschlagt ist. Das Biogas sammelt sich im oberen Abschnitt 14 des Außenbehälters 12, wie durch Punkte in FIG. 2 dargestellt. Da die Oberkante der Innenröhre 26 höher ist, als die Überlaufkante 22, wird vermieden, dass die Gärmasse sich direkt von der Einfüllkammer 20 in die zweite Zwischenkammer 32 bewegt. Vielmehr bewegt sich die in die erste Zwischenkammer 30 eingefüllte Gärmasse in dieser nach unten. Diese Bewegung der Gärmasse nach unten wird durch die nachgefüllte Gärmasse vorangetrieben. Im unteren Bereich des Innenbehälters 18 ist die erste Zwischenkammer 30 mit der zweiten Zwischenkammer 32 verbunden, so dass die Gärmasse, welche am unteren Ende aus der ersten Zwischenkammer 30 austritt, in das untere Ende der zweiten Zwischenkammer 32 eintritt. In dieser zweiten Zwischenkammer 32 steigt die Gärmasse nach oben. Durch das Prinzip kommunizierender Gefäße herrscht in der ersten Zwischenkammer 30 und in der zweiten Zwischenkammer 32 im Wesentlichen der gleiche Füllstandspegel. Dieser Füllstandspegel entspricht der Einfüllöffnung 34. Erreicht die Gärmasse in der zweiten Zwischenkammer 32 die Einfüllöffnung 34, so stürzt die Gärmasse in den Rücklaufkanal 36 und sinkt in diesem nach unten. Die Höhe, um welche das Substrat im Rücklaufkanal 36 nach unten stürzt hängt mitunter vom Druck ab, mit dem der Innenraum des Biogasreaktors 10 beaufschlagt ist. Auch beim Stürzen von der zweiten Zwischenkammer 32 in den Rücklaufkanal 36 wird die Biomasse vollständig entgast. Dadurch, dass der Ablaufkanal 42 mit dem Rücklaufkanal 36 eine kommunizierende Röhre ausbildet, hängt der Füllstandspegel im Ablaufkanal 42 vom Füllstandspegel im Rücklaufkanal 36 ab. Etwa die Hälfte des im Rücklaufkanal 36 sich nach unten bewegenden Substrats verlässt den Biogasreaktor über den Ablaufkanal 42 und die andere Hälfte wird durch die Impfpumpe 38 in die Mischeinheit 86 gefördert, wo sie sich mit dem neu zugeführten Frischsubstrat, wie vorstehend erläutert, vermischt. Innerhalb des Außenbehälters 12 wird die Biomasse durch die Heizung 62 auf einer Temperatur von ca. 35°C gehalten. Das vorliegende Verfahren läuft im mesophilen Bereich (30°C-38°C) ab, weil in diesem Bereich die Abbauraten höher sind und somit eine größere Gasmenge erzeugt werden kann. Die im Verfahren vorliegenden Methanbakterien sind sehr empfindlich und benötigen möglichst eine gleichbleibende Temperatur, die keinen starken Schwankungen unterworfen ist.

Der Rücklaufkanal 36 wird nach etwa 8 bis 10 Tagen erreicht wobei das Substrat an dieser Position nur noch reaktionslose Gärmasse beinhaltet, welche mit den vorherrschenden Gärbakterien hoch angereichert ist. Das sich im oberen Abschnitt 14 des Außenbehälters 12 angesammelte Biogas wird mittels des Flüssigkeitsbehälters 66 auf einem konstanten Druck gehalten und zunächst über die Gasabführleitung 64 in den Flüssigkeitsbehälter 66 kontinuierlich abgeführt und von dort über die Gaseinspeisleitung 68 zu einem nicht dargestellten Gasspeicher eingespeist. Dabei werden keine Druckventile eingesetzt, welche leicht verschleißen würden, sondern der Druck wird über die Eintauchtiefe von 1 m bis 2 m konstant gehalten. Die Förderung der Gärmasse im ganzen System erfolgt mittels Pumpendruck der Pumpen 38 und 92, durch das hydrostatische Gefälle von der Überlaufkante 22 zur Einfüllöffnung 34 und durch den Gasdruck im oberen Abschnitt 14 von 0,1 bis 0,2 bar. Über das statische Gefälle sowie den Innendruck kann die Förderwirkung für die im Biogasreaktor befindlichen organischen Stoffe variiert werden, d.h. das statische Gefälle und/oder der Innendruck können auf die Konsistenz der organischen Stoffe abgestimmt werden.

Im Ergebnis wird ein Gärprozess erlangt, in dem organische Bestandteile in verhältnismäßig kurzer Zeit, etwa 6 bis 10 Tage, bis zu 70% und mehr reduziert werden. Das bedeutet die Gewinnung einer großen Gasmenge und bei entsprechenden Rahmenbedingungen eine sehr gute Wirtschaftlichkeit. Die Umwandlung der organischen Stoffe findet in geschlossenen Behältern statt, so dass keine Gerüche nach außen austreten. Die Gärreststoffe, die am Ablaufrohr 40 austreten, haben einen erdigen Geruch und sind frei von Schleimstoffen und in jeder Hinsicht umweltverträglich. Diese können ohne weitere Behandlung als Naturdünger auf landwirtschaftliche Flächen ausgebracht werden. Sollte eine Weiterbehandlung aus zwingenden Gründen erforderlich sein, so ist eine Fest-Flüssigtrennung angebracht und mit geringem Aufwand möglich, weil sich die ausgegorenen Reststoffe leicht trennen lassen.

Die erlangte Biogasmenge ist mit ca. 2 m³ Biogas pro Kilogramm abgebauter organischer Trockensubstanz im Bereich des Erreichbaren. Das Biogas weist einen Methananteil von etwa 70 - 74% auf, wodurch ein Energiewert von 7,0 - 7,4 kWh/m³ Biogas erreicht wird. Ein weiterer nicht zu unterschätzender Vorteil der vorstehend beschriebenen Vorrichtung beziehungsweise dessen zugehörigen Verfahrens liegt darin, dass Substrate mit Trockensubstanzanteilen von 20 - 25% breiig aber noch pumpfähig vergärt werden können. Selbst bei extrem hohen Trockensubstanzanteilen gibt es keine Strömungsprobleme im Biogasreaktor 10, da Zusammenmischung von reaktionsloser Gärmasse und Frischsubstrat bei der Zusammenmischung vor Eintritt in den Gärbehälter durch die reaktionslose Gärmasse eine Verdünnung erfährt und das Substrat auf einen für Biozönose bevorzugten pH-Wert von ca. 7,5 bis 7,8 anhebt. Die vorstehend beschriebene Vorrichtung und das vorstehend beschriebene Verfahren gewährleisten den Abbau der organischen Stoffe von über 90%. Somit ist eine optimale Ausgärung erreicht und die Reststoffe (die Impfmasse) haben einen pH-Wert, der größer als 8,0 bis 8,4 ist. Dies hat zur Folge, dass die Rückführrate (die Menge an Impfmasse) direkten Einfluss auf das Substrat hat. Frischsubstrat und Impfmasse im richtigen Verhältnis, vornehmlich im Verhältnis 1:1, innig vermischt bilden das Gärsubstrat. In diesem Gärsubstrat stellt sich ein pH-Wert ein, der größer als 7,0 ist, und liefert die Grundlagen und die Voraussetzungen für eine sehr gut ablaufende Biozönose. Insgesamt können mit dem vorstehend beschriebenen Verfahren gewaltige wirtschaftliche Vorteile erzielt werden, hinzu kommen die Vorteile einer Werksfertigung und der Vorteil kleinere Anlagen zu fertigen und einen weitaus größeren Marktbereich zu erfassen.

Ferner sind folgende Vorteile durch die beschriebene Vorrichtung und das beschriebene Verfahren erreichbar:
- Durch die ausgegorene Impfmasse wird die größtmögliche Menge lebensfähiger, anaerober Bakterien in das Frischsubstrat eingetragen, also eine starke Kultur, die den Gärprozess stürmisch beginnen lässt und die starke Aktivität auf unbegrenzte Zeit anhält.
- Durch die Anhebung des für die Biozönose günstigen pH-Werts auf über 7,0 wird ein Mileu geschaffen, das den Gärprozess optimal und ungestört ablaufen lässt.
- Dadurch, dass die Rückführrate (Impfmenge) exakt gesteuert werden kann, werden Substrate mit niederem pH-Wert von weniger als 7,0 über die Impfmenge aufgewertet. So kann es durchaus erforderlich sein, die Impfmenge zu erhöhen und so von vornherein ein der Biozönose in einer anaeroben Symbiose günstiges Milieu zu schaffen.

Allgemein bestehen organische Reststoffe aus den drei Grundbestandteilen: Kohlehydrate, organische Fette und Eiweißstoffe. Diese drei Grundbestandteile spielen in der anaeroben Vergärung eine wichtige Rolle und beinhalten ein bestimmtes Potenzial an Energie. Durch wissenschaftliche Untersuchungen konnte ermittelt werden, dass aus den drei Grundbestandteilen bezogen auf die abgebaute organische Trockensubstanz (OTS) nachfolgende Biogasmengen mit der entsprechenden Qualität gewonnen werden können.

| | |
|---|---|
| Kohlenhydrate ergeben | 790cm³/kg mit ca. 50% CH₄ + ca. 50% CO₂ |
| Organische Fette | 1.250cm³/kg mit ca. 68% CH₄ + ca. 32% CO₂ |
| Eiweißstoffe | 704cm³/kg mit ca. 71% CH₄ + ca. 29% CO₂ |
| Biogas | ca. 2.744m³/kg abgebauter OTS |

Im vorliegenden Gärprozess sind höchste Abbauraten der organischen Trockensubstanz möglich, wozu wesentlich die hohe Rückführrate (Impfmasse) beiträgt. 50% der Gärmasse durchläuft den Prozess zweimal. Doch auch ein normaler Durchlauf von 10 Tagen garantiert eine optimale Abbaurate der organischen Masse. Der Anaerobprozess der vorliegenden Erfindung ist in ca. 6 bis 10 Tagen abgeschlossen. Mit diesem Verfahren können ausgehend von einer Abbaurate von ca. 70% OTS 1,920 m³ Biogas pro kg abgebauter OTS erreicht werden, die einen Anteil von ca. 68 - 74% CH₄ enthalten, was 7,1 kWh/m³ Biogas ergibt.

Es sei noch erwähnt, dass die Faulraumbelastung ein Begriff ist, der aus der Klärtechnik in die Vergärung übernommen wurde. Bedeutung hat sie nur da, wo im Reaktor gerührt und gemischt wird und ist daher für das vorliegende Verfahren unwesentlich. Werte von ca. 6,0 - 6,5 kg OTS/m³ Faulraum beeinträchtigen den Gärprozess des vorliegenden Verfahrens nicht, wohingegen in Verfahren vom Stand der Technik maximale Werte von ca. 1,5 kg OTS/m³ Faulraum bis 2,5 kg OTS/m³ Faulraum genannt werden.

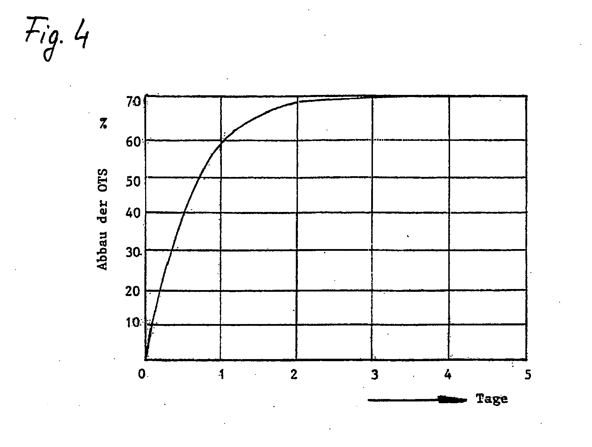

FIG. 4 zeigt einen Verlauf der Vergärung, den das Verfahren der vorliegenden Erfindung sicher erreicht. Der in dieser Figur dargestellte Ablauf des Abbaus der organischen Trockensubstanz (OTS) wird durch das Verfahren der vorliegenden Erfindung sicher erreicht.

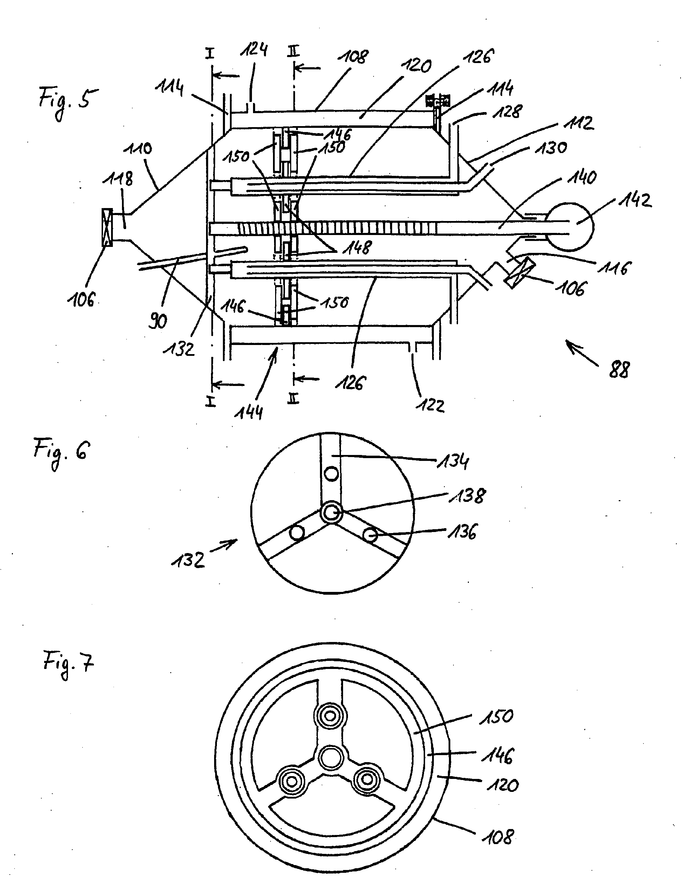

FIG. 5 zeigt eine schematische Schnittdarstellung eines Wärmetauschers der Vorrichtung aus FIG. 2. Das Gehäuse des Wärmetauschers 88 umfasst im mittleren Bereich einen liegenden, doppelwandigen Abschnitt 108, der beidseitig mit nach außen hin verjüngten Abschlüssen 110, 112 versehen ist. Der doppelwandige Abschnitt 108 ist vorzugsweise zylinderförmig und der linke und rechte Abschluss 110, 112 entsprechend trichterförmig, jedoch sind alternativ auch andere Querschnittsformen wie beispielsweise ein viereckiger Querschnitt möglich. Der doppelwandige Abschnitt 108 des Wärmetauschergehäuses ist an beiden Enden über einen Lochflansch 114 mit dem linken und rechten Abschluss 110, 112 verbunden. Der rechte Abschluss 112 ist an seiner unteren Flanke mit einem Rohrstutzen 116 versehen, der mit der Frischsubstrat-Pumpe 92, beispielsweise mittels eines Lochflansches, verbindbar ist. Der linke Abschluss 110 des Wärmetauschergehäuses ist mit einem Rohrstutzen 118 versehen, der beispielsweise mittels eines Lochflansches mit der Mischeinheit 86 verbindbar ist. Der Raum des doppelwandigen Abschnittes 108 des Wärmetauschergehäuses, der durch die Doppelwandung ausgebildet wird, dient als Wärmeträgerraum 120, der eine Zuleitung 122 zum Einleiten eines heißen Fluids, vorzugsweise Wasser, und eine Ableitung 124 zum Abführen des Fluids aufweist. Das Fluid, mit der der Wärmeträgerraum 120 durchströmt werden kann, kann beispielsweise in einem Blockkraftwerk erwärmt werden. Die Temperatur des Fluids an der Zuleitung 122 beträgt.vorzugsweise 80°C. Der doppelwandige Abschnitt 108 bildet somit einen Wärmeeintragungskörper, mit dem organisches Frischsubstrat vorgewärmt werden kann, das über den Rohrstutzen 116 in den Innenraum des Wärmetauschers 88 zugeführt wird, und über den Rohrstutzen 118 nach dem Vorwärmen abführbar ist. Der Betrieb des Wärmetauschers 88 unter Einbindung des Temperatursensors 90 wurde bereits im Zusammenhang mit FIG. 2 beschrieben. Des Weiteren sind drei doppelwandige Wärmeträgerrohre 126 im Innenraum des Wärmetauschers 88 angeordnet, die so ausgebildet sind, dass ein Vorlauf einen Rücklauf umgibt. Über einen Einlass 128 des Vorlauf ist somit das selbe Fluid wie in den Wärmeträgerraum 120 zuführbar, welches das Wärmeträgerrohr 126 im Vorlauf von rechts nach links im äußeren Rohrmantel durchläuft und am linken Ende des Wärmeträgerrohrs 126 im Mittelteil des Wärmeträgerrohrs 126 von links nach rechts im Rücklauf zu einem Auslass 130 des Rücklaufs zurückgeführt wird. Die drei Wärmeträgerrohre 126 dienen somit ebenfalls als Wärmeeintragungskörper zum Vorwärmen des im Innenraum des Wärmetauschers 88 befindlichen Substrats auf vorzugsweise 35 - 37°C. Rechtsseitig werden die Wärmeträgerrohre 126 durch den rechten Abschluss 112 des Wärmetauschergehäuses fixiert. Linksseitig werden die drei Wärmeträgerrohre 126 durch eine Stützscheibe 132 fixiert.

FIG. 6 zeigt eine schematische Schnittdarstellung entlang der Linie I-I aus FIG. 5. Die Stützscheibe 132 weist drei sternförmig von der Mitte zum Randbereich verlaufende Rippen 134 auf, in denen jeweils mittig ein Aufnahmeloch 136 ausgebildet ist. Die Aufnahmelöcher 136 sind als Sacklöcher ausgebildet und dienen zur Aufnahme und zur Abstützung der linken Enden der Wärmeträgerrohre 126. Ein in der Mitte der Stützscheibe 132 ausgebildetes Loch ist ebenfalls als Sackloch ausgebildet und mit einem Lager 138 versehen, so dass das linke Ende einer Spindel 140 darin gelagert werden kann.

Erneut unter Bezugnahme auf FIG. 5 verläuft die Spindel 140 mittig im Wärmetauscher 88 in Längsrichtung des Wärmetauschers 88. Der Abschnitt der Spindel 140, welcher parallel zu den Wärmeträgerrohren 126 verläuft, ist mit einem Gewinde versehen, Das rechte Ende der Spindel 140 ist aus dem rechten Abschluss 112 des Wärmetauschergehäuses herausgeführt und mit einer Antriebseinheit 142 verbunden. Im Betrieb des Wärmetauschers 88 flockt bei der Vorwärmung dickflüssiger Biomasse im Wärmetauscher 88 Eiweiß ab ca. 60°C aus und setzt sich an den Flächen der Wärmeeintragungskörper 108, 126 ab. Zur Entfernung dieser Anhaftungen ist eine Reinigungseinrichtung 144 im Wärmetauscher 88 vorgesehen. Die Reinigungseinrichtung 144 umfasst eine Reinigungsscheibe 146 deren Außendurchmesser minimal kleiner als der Innenndurchmesser des doppelwandigen Abschnittes 108 ist. Dabei bedeutet die Bezeichnung "minimal kleiner", dass nur soviel Spiel zwischen den Elementen ausgebildet ist, wie für eine Relativbewegung zueinander erforderlich ist. Außerdem sind drei Reinigungsscheiben 148 vorgesehen, von denen jede jeweils ein Wärmeträgerrohr 126 umgibt, wobei der Außendurchmesser der Wärmeträgerrohre 126 minimal kleiner als Innendurchmesser der Reinigungsscheiben 148 ist. Der Außendurchmesser der Reinigungsscheiben 148 und der Innendurchmesser der Reinigungsscheibe 146 sind so dimensioniert, dass im montierten Zustand ein Abstand zwischen dem Innendurchmesser der Reinigungsscheibe 146 und dem Außendurchmesser der Reinigungsscheiben 148 sowie zwischen dem Außendurchmesser der Reinigungsscheiben 148 und der Spindel 140 ausgebildet wird. Die Reinigungsscheiben 146 und 148 sind zwischen zwei Reinigungsstützscheiben 150 angeordnet.

FIG. 7 zeigt eine schematische Schnittdarstellung entlang der Linie II-II aus FIG. 5. Die Reinigungsstützscheiben haben einen ringförmigen Abschnitt, dessen Außendurchmesser kleiner als der Innendurchmesser des doppelwandigen Abschnittes 108 ist. Von diesem ringförmigen Abschnitt erstrecken sich drei Rippen nach innen, wobei am Schnittpunkt der drei Rippen in der Mitte der Reinigungsstützscheibe 150 ein Gewinde entsprechend zum Gewinde der Spindel 140 ausgebildet ist. Jeweils mittig ist jede der drei Rippen mit einem Ringabschnitt versehen, d. h. in diesem Bereich umgibt die Rippe im montierten Zustand ringartig ein Wärmeträgerrohr 126. Dabei ist der Innendurchmesser dieses ringartigen Abschnittes der Rippen größer als der Außendurchmesser der Wärmeträgerrohre 126.

Erneut unter Bezugnahme auf FIG. 5 werden mit den wie vorstehend beschrieben aufgebauten Reinigungsstützscheiben 150 die Reinigungsscheiben 146 und 148 beidseitig gestützt und geführt.

Zur Reinigung der Wärmeeintragungskörper 108, 126 von Anhaftungen wird im Betrieb des Wärmetauschers 88 die Antriebseinheit 142 in eine Richtung bewegt (beispielsweise Rechts- oder Linkslauf eines Elektromotors), so dass über den Gewindeeingriff zwischen Spindel 140 und Reinigungsstützscheibe 150 die gesamte Reinigungseinrichtung 144 in eine Richtung bewegt wird, da die Wärmeträgerrohre 126 eine Drehung der Reinigungseinrichtung 144 mit der Spindel 140 verhindern. Dabei streifen die Reinigungsscheiben 146 über die Wärmeträgerrohre 126 und die Reinigungsscheiben 148 über die Innenseite des doppelwandigen Abschnittes 108. Bei Erreichen einer Begrenzung wird die Antriebseinheit 142 umgeschaltet, so dass sie die Spindel 140 mit entgegengesetztem Drehsinn dreht. Somit wird die Reinigungseinrichtung 144 in die entgegengesetzte Richtung bewegt, bis zum erneuten Erreichen einer Begrenzung. Diese Begrenzung kann sowohl eine zeitliche Begrenzung sein, als auch mittels Sensoren erfasst werden. Die Begrenzung entspricht im Wesentlichen dem rechten und linken Ende der Wärmeträgerrohre 126, dem rechten und linken Ende des Gewindeganges auf der Spindel 140 und dem rechten und linken Ende des doppelwandigen Abschnittes 108. Durch den Spindelantrieb kann eine Hin- und Herbewegung der Reinigungseinrichtung erreicht werden, mit der die Wärmeeintragungskörper 108, 126 von Anhaftungen befreit werden. Die abgeschabten Anhaftungen gelangen in den regulären Substratfluss, da sie zwangsläufig in den linken Abschluss 110 des Wärmetauschergehäuses gedrückt und mit jedem Pumpzyklus in das Substrat untergemischt werden. Außerdem wird durch die Hin- und Herbewegung der Reinigungseinrichtung 144 eine ständige Durchmischung des Frischsubstrates erreicht, so dass eine gleichmäßige Vorwärmung des Frischsubstrates gewährleistet ist.

Der linke und rechte Abschluss 110 und 112 können mit wenigen Handgriffen vom doppelwandigen Abschnitt 108 entfernt werden, so dass der Wärmetauscher 88 leicht von innen inspiziert werden kann.

Alternativ zur Spindel 140 kann zur Bewegung der Reinigungseinrichtung 144 auch ein elektrohydraulisch angetriebener Zylinder oder ein pneumatisch angetriebener Zylinder vorgesehen sein.

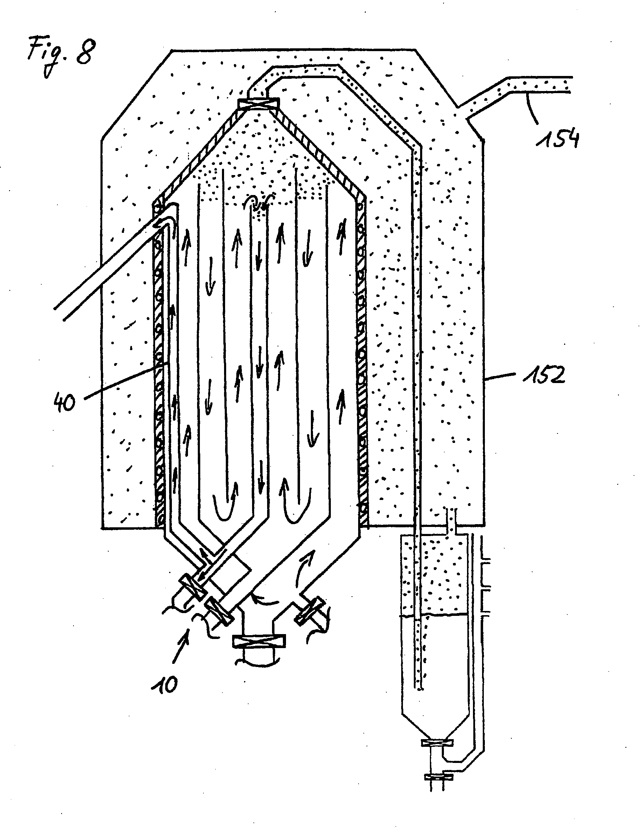

Fig. 8 zeigt eine schematische Darstellung einer Weiterbildung der erfindungsgemäßen Vorrichtung zur Erzeugung von Biogas. Die Vorrichtung aus Fig. 8 unterscheidet sich von der vorstehend beschriebenen Vorrichtung dadurch, dass zusätzlich ein Gasspeicher 152 vorgesehen ist. Der Gasspeicher 152 umgibt den Biogasreaktor 10 so, dass lediglich der untere Abschnitt 16 des Außengehäuses freigelegt bleibt. Der Auslass des Ablaufrohrs 40 ist in dieser Weiterbildung etwas verlängert, so dass der Auslass durch den Gasbehälter 152 hindurch geführt werden kann. Der Flüssigkeitsbehälter 66 ist in dieser Weiterbildung nach unten versetzt, so dass der Flüssigkeitsbehälter 66 unterhalb des Gasbehälters 152 angeordnet ist. Das im Biogasreaktor 10 erzeugte Biogas verlässt den Biogasreaktor und führt über die im Gasbehälter 152 verlaufende Gasabführleitung 64 zum Flüssigkeitsbehälter 66. Das den Flüssigkeitsbehälter 66 verlassende Biogas wird dem Gasspeicher 152 zugeführt. Über eine Gaseinspeisleitung 154 kann das Biogas dem Gaspeicher 152 entnommen werden.

Alternativ zum beschriebenen Ausführungsbeispiel ist es möglich, bei der in FIG. 2 dargestellten Vorrichtung zur Erzeugung von Biogas den Wärmetauscher 88 entfallen zu lassen und das Frischsubstrat mittels Dampfeintragung vorzuwärmen.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

Ferner behält sich der Anmelder ausdrücklich das Recht vor, folgende Gegenstände im Rahmen dieser Erfindung zu beanspruchen:

Einen Wärmetauscher für eine Biogasanlage, mit Wärmeeintragungskörpern 108, 126 zum Erwärmen einer im Wärmetauscher 88 befindlichen Masse mittels räumlich davon getrenntem Fluid, der gekennzeichnet ist, durch eine Reinigungseinrichtung 144, die bei geschlossenem Wärmetauscher 88 über die Wärmeeintragungskörper 108, 126 streifbar ist.

Einen weitergebildeten Wärmetauscher der, dadurch gekennzeichnet ist, dass die Reinigungseinrichtung 144 mittels eines Spindelantriebs 140 hin und her bewegbar ist.

Einen weitergebildeten Wärmetauscher der, dadurch gekennzeichnet ist, dass die Wärmeeintragungskörper 108, 126 doppelwandige Rohre, sind in denen der Vorlauf den Rücklauf ummantelt oder der Rücklauf den Vorlauf ummantelt.

### Bezugszeichenliste:

- 10: Biogasreaktor
- 12: Außengehäuse
- 14: Oberer Abschnitt des Außengehäuses
- 16: Unterer Abschnitt des Außengehäuses
- 18: Innengehäuse
- 20: Einfüllkammer
- 22: Überlaufkante
- 24: Unterer Abschnitt des Innenbehälters
- 26: Innenröhre
- 28: Rücklaufrohr
- 30: Erste Zwischenkammer
- 32: Zweite Zwischenkammer
- 34: Einfüllöffnung
- 36: Rücklaufkanal
- 38: Impfpumpe
- 40: Ablaufrohr
- 42: Ablaufkanal
- 44: Rohrstück
- 46: Rohrstück
- 48: Schieber
- 50: Schieber
- 52: Schieber
- 54: Schieber
- 56: Sammelraum
- 58: Sammelraum
- 60: Isolation
- 62: Heizung
- 64: Gasabführleitung
- 66: Flüssigkeitsbehälter
- 68: Gaseinspeisleitung
- 70: Rohrstück
- 72: Steigrohr
- 74: Öffnungen
- 76: Flüssigkeit
- 78: Ende der Gasabführleitung
- 80: Schieber
- 82: Schieber
- 84: Sammelraum
- 86: Mischeinheit
- 88: Wärmetauscher
- 90: Temperatursensor
- 92: Frischsubstrat-Pumpe
- 94: Zerkleinerungseinheit
- 96: Schneidewerkzeug
- 98: Lochscheibe
- 100: Welle
- 102: Einfülltrichter
- 104: Antriebseinheit
- 106: Schieber
- 108: Doppelwandiger Abschnitt des Wärmetauschergehäuses
- 110: Linker Abschluss des Wärmetauschergehäuses
- 112: Rechter Abschluss des Wärmetauschergehäuses
- 114: Lochflansch
- 116: Rohrstutzen
- 118: Rohrstutzen
- 120: Wärmeträgerraum
- 122: Zuleitung
- 124: Ableitung
- 126: Wärmeträgerrohr
- 128: Einlass
- 130: Auslass
- 132: Stützscheibe
- 134: Rippe
- 136: Aufnahmeloch
- 138: Lager
- 140: Spindel
- 142: Antriebseinheit
- 144: Reinigungseinrichtung
- 146: Reinigungsscheibe
- 148: Reinigungsscheibe
- 150: Reinigungsstützscheibe
- 152: Gasspeicher
- 154: Gaseinspeisleitung

## Patentansprüche

1. Vorrichtung zur Erzeugung von Biogas aus organischen Stoffen, mit einem Biogasreaktor (10), der eine Einfüllkammer (20) zur Befüllung mit den organischen Stoffen und einen Rücklaufkanal (36) zum zumindest teilweisen Abführen der organischen Stoffe aus dem Biogasreaktor (10) aufweist, **dadurch gekennzeichnet, dass** der Biogasreaktor (10) des Weiteren zumindest eine erste Zwischenkammer (30) und eine zweite Zwischenkammer (32) aufweist, wobei die Einfüllkammer (20), die erste Zwischenkammer (30), die zweite Zwischenkammer (32) und der Rücklaufkanal (36) in dieser Reihenfolge Abschnitte einer nur in eine Richtung durchströmbaren Strömungsbahn für die organischen Stoffe ausbilden, wobei von zwei aufeinander folgenden Abschnitten jeweils einer eine ansteigende Strömungsbahn und der andere eine abfallende Strömungsbahn ausbildet, dass die Einfüllkammer (20) eine Überlaufkante (22) aufweist, mittels der die erste Zwischenkammer (30) von der Einfüllkammer (20) aus mit den organischen Stoffen befüllbar ist, dass die erste Zwischenkammer (30) und die zweite Zwischenkammer (32) in einem Bereich unterhalb einer Einfüllöffnung (34) des Rücklaufkanals (36) und oberhalb der Überlaufkante (22) in Verbindung stehen, und dass die erste Zwischenkammer (30) und die zweite Zwischenkammer (32) im Innenraum eines becherartigen Innenbehälters (18) ausgebildet sind, der im Innenraum des Biogasreaktors (10) angeordnet ist.

2. Vorrichtung gemäß Anspruch 1, **dadurch gekennzeichnet, dass** von dem Rücklaufkanal (36) ein Ablaufkanal (42) derart abzweigt, dass der Ablaufkanal (42) mit dem Rücklaufkanal (36) eine kommunizierende Röhre ausbildet.

3. Vorrichtung gemäß Anspruch 1 oder 2, **dadurch gekennzeichnet, dass** die Querschnittsfläche der Einfüllkammer (20) im Bereich der Überlaufkante (22) verengt ist.

4. Vorrichtung gemäß einem der vorhergehenden Ansprüche, **dadurch gekennzeichnet, dass** der Biogasreaktor (10) einen sich nach oben verjüngenden oberen Abschnitt (14) aufweist und die Überlaufkante (22) sich so weit in den oberen Abschnitt (14) erstreckt, dass die Querschnittsfläche der Einfüllkammer (20) im Bereich der Überlaufkante (22) verengt wird.

5. Vorrichtung gemäß einem der vorhergehenden Ansprüche **dadurch gekennzeichnet, dass** der Biogasreaktor (10) einen sich nach unten verjüngenden unteren Abschnitt (16) aufweist, wobei im unteren Bereich des unteren Abschnittes (16) ein Sammelraum (56) für Sedimente ausgebildet ist, der einerseits wahlweise mit dem Innenraum des Biogasreaktors (10) verbindbar ist und andererseits wahlweise mit der Umgebung des Biogasreaktors (10) verbindbar ist.

6. Vorrichtung gemäß einem der vorhergehenden Ansprüche, **dadurch gekennzeichnet, dass** im oberen Bereich des Biogasreaktors eine Gasabführleitung (64) angeschlossen ist, über welche der Innenraum des Biogasreaktors (10) mit einem konstanten Druck beaufschlagbar ist.

7. Vorrichtung gemäß Anspruch 6, **dadurch gekennzeichnet, dass** des Weiteren ein Flüssigkeitsbehälter (66) vorgesehen ist, der mit einer Flüssigkeit (76) befüllbar ist, wobei das vom Innenraum des Biogasreaktors (10) herausführende Ende (78) der Gasabführleitung (64) in der Flüssigkeit (76) platzierbar ist, so dass über die Eintauchtiefe in die Flüssigkeit (76), der Druck im Innenraum des Biogasreaktors (10) einstellbar ist.

8. Vorrichtung gemäß einem der vorhergehenden Ansprüche, **dadurch gekennzeichnet, dass** des Weiteren eine Mischeinheit (86) zur Befüllung der Einfüllkammer (20) vorgesehen ist, die dazu angepasst ist, im Wesentlichen in einem 1:1 Verhältnis, neue organische Stoffe und mittels Rückführkanal (36) aus dem Biogasreaktor (10) abgeführte organische Stoffe zu mischen.

9. Vorrichtung gemäß einem der vorhergehenden Ansprüche, **dadurch gekennzeichnet, dass** des Weiteren ein Wärmetauscher (88) vorgesehen ist, der stromaufwärts der Einfüllkammer (20) angeordnet ist und dazu angepasst ist, die neu zuzuführenden organischen Stoffe mittels eines heißen Fluids vorzuwärmen.

10. Vorrichtung gemäß einem der vorhergehenden Ansprüche, **dadurch gekennzeichnet, dass** eine Zerkleinerungseinheit (94) stromaufwärts der Einfüllkammer (20) vorgesehen ist.

11. Vorrichtung gemäß einem der vorhergehenden Ansprüche, **dadurch gekennzeichnet, dass** ein Gasspeicher (152) vorgesehen ist, in dem im Biogasreaktor (10) erzeugtes Biogas speicherbar ist und der den Biogasreaktor (10) zumindest teilweise umgibt.

12. Verfahren zur Biogaserzeugung mit den Schritten:
- Einfüllen organischer Stoffe in eine Einfüllkammer (20) eines Biogasreaktors (10);
- Unidirektionales Fördern der organischen Stoffe entlang der Abschnitte gebildet durch die Einfüllkammer (20), zumindest eine erste Zwischenkammer (30) sowie eine zweite Zwischenkammer (32), die im Innenraum eines in dem Biogasreaktor (10) angeordneten becherartigen Innenbehälters (18) ausgebildet sind, und einen Rücklaufkanal (36), wobei von zwei aufeinander folgenden Abschnitten jeweils einer eine ansteigende Strömungsbahn und der andere eine abfallende Strömungsbahn ausbilden;
- Ausgasen der organischen Stoffe durch zeitweiliges Freilegen der organischen Stoffe beim Übergang von der Einfüllkammer (20) in die erste Zwischenkammer (30).

13. Verfahren gemäß Anspruch 12, **dadurch gekennzeichnet, dass** die innerhalb des Biogasreaktors (10) ablaufenden Verfahrensschritte ohne aktives Umrühren der organischen Stoffe ausgeführt werden.

14. Verfahren gemäß einem der Ansprüche 12 oder 13, **dadurch gekennzeichnet, dass** die neu zugeführten organischen Stoffe vor dem Einfüllen in die Einfüllkammer (20) mittels eines Wärmetauschers (88) auf 35°C - 37°C vorgewärmt werden.

15. Verfahren gemäß einem der Ansprüche 12 bis 14, **dadurch gekennzeichnet, dass** die in die Einfüllkammer (20) eingefüllten organischen Stoffe aus neu zugeführten vorgewärmten organischen Stoffen und direkt mittels Rücklaufkanal (36) abgeführten organischen Stoffen vermischt werden.

16. Verfahren gemäß Anspruch 15, **dadurch gekennzeichnet, dass** das Mischungsverhältnis 1:1 beträgt.

17. Verfahren gemäß einem der Ansprüche 12 bis 16, **dadurch gekennzeichnet, dass** im Innenraum des Biogasreaktors (10) ein Innendruck konstant gehalten wird.

18. Verfahren gemäß einem der Ansprüche 12 bis 17, **dadurch gekennzeichnet, dass** die organischen Stoffe über eine Überlaufkante (22) der Einfüllkammer (20) in die zumindest eine Zwischenkammer (30) von der Einfüllkammer (20) aus füllbar sind, und die organischen Stoffe im Bereich der Überlaufkante (22) mittels einer Querschnittsflächenverengung der Einfüllkammer (20) verdichtet werden.

## Claims

1. A device for generating biogas from organic materials comprising a biogas reactor (10) provided with a charging chamber (20) to be charged with the organic materials and a backflow channel (36) for at least partly discharging the organic materials from the biogas reactor (10), **characterised in that** the biogas reactor (10) further comprises at least a first intermediate chamber (30) and a second intermediate chamber (32), the charging chamber (20), the first intermediate chamber (30), the second intermediate chamber (32), and the backflow channel (36) forming, in the named order, parts of a flow path for the organic materials which can be passed in only one direction, one of the two portions succeeding each other respectively forming a rising flow path while the other forms a falling flow path, wherein the charging chamber (20) comprises an overflow rim (22) by means of which the intermediate chamber (30) can be charged with the organic materials from the charging chamber (20), the first intermediate chamber (30) and the second intermediate chamber (32) being connected in a section below a charging orifice (34) of the backflow channel (36) and above the overflow rim (22), and wherein the first intermediate chamber (30) and the second intermediate chamber (32) are formed in the interior of a cup-like interior reservoir (18) arranged in the interior of the biogas reactor (10).

2. The device according to claim 1, **characterised in that** a discharge channel (42) branches off the backflow channel (36) so that the discharge channel (42) forms a communicating pipe together with the backflow channel (36).

3. The device according to claim 1 or 2, **characterised in that** the cross sectional area of the charging chamber (20) is narrowed in the area of the overflow rim (22).

4. The device according to one of the preceding claims, **characterised in that** the biogas reactor (10) comprises an upper portion (14) tapered towards the upper side and **in that** the overflow rim (22) extends so far into the upper portion (14) that the cross sectional area of the charging chamber (20) is narrowed in the area of the overflow rim (22).

5. The device according to one of the preceding claims **characterised in that** the biogas reactor (10) comprises a lower portion (16) tapered towards the bottom, a receptacle (56) for sediments being formed in the lower section of the lower portion (16) which, on the one hand, is optionally connectable to the interior of the biogas reactor (10) and, on the other hand, optionally connectable to the surroundings of the biogas reactor (10).

6. The device according to one of the preceding claims, **characterised in that** a gas discharge line (64) is connected in the upper section of the biogas reactor via which a constant pressure can be applied to the interior of the biogas reactor (10).

7. The device according to claim 6, **characterised in that** furthermore a liquid reservoir (66) is provided which can be charged with a liquid (76), the end (78) of the gas discharge line (64) leading out of the interior of the biogas reactor (10) being placable in the liquid (76) so that the pressure in the interior of the biogas reactor (10) is adjustable via the immersion depth in the liquid (76).

8. The device according to one of the preceding claims, **characterised in that** furthermore a mixing unit (86) for charging the charging chamber (20) is provided which is adjusted to mix new organic materials and organic materials discharged from the biogas reactor (10) by means of a recirculation conduit (36) in a ratio of substantially 1:1.

9. The device according to one of the preceding claims, **characterised in that** furthermore a heat exchanger (88) disposed upstream of the charging chamber (20) and adjusted to pre-heat the organic materials to be newly supplied by means of a hot fluid is provided.

10. The device according to one of the preceding claims, **characterised in that** a crushing unit (94) is provided upstream of the charging chamber (20).

11. The device according to one of the preceding claims, **characterised in that** a gasholder (152) is provided in which biogas generated in the biogas reactor (10) is storable and which at least partly surrounds the biogas reactor (10).

12. A process for generating biogas comprising the steps of:
- charging a charging chamber (20) of a biogas reactor (10) with organic substances;
- unidirectionally delivering the organic materials along the portions formed by the charging chamber (20), at least a first intermediate chamber (30) and a second intermediate chamber (32) which are formed in the interior of a cup-like interior reservoir (18) arranged in the interior of the biogas reactor (10), and a backflow channel (36), two subsequent portions respectively forming a rising flow path and a falling flow path;
- degassing of the organic materials by means of a temporary exposure of the organic materials during the transition from the charging chamber (20) into the first intermediate chamber (30).

13. The process according to claim 12, **characterised in that** the process steps taking place inside of the biogas reactor (10) are carried out without an active stirring of the organic materials.

14. The process according to one of the claims 12 or 13, **characterised in that** the newly supplied organic materials are pre-heated to 35°C - 37°C by means of a heat exchanger (88) before being filled into the charging chamber (20).

15. The process according to one of the claims 12 to 14, **characterised in that** the organic materials with which the charging chamber (20) is charged are mixed from newly supplied, pre-heated organic materials and organic materials directly discharged by means of the backflow channel (36):

16. The process according to claim 15, **characterised in that** the mixture ratio is 1:1.

17. The process according to one of the claims 12 to 16, **characterised in that** an internal pressure is kept constant in the interior of the biogas reactor (10).

18. The process according to one of the claims 12 to 17, **characterised in that** the organic materials can be filled into the at least one intermediate chamber (30) from the charging chamber (20) via an overflow rim (22) of the charging chamber (20) and **in that** the organic materials are densified in the area the overflow rim (22) by means of a narrowing of the cross sectional area of the charging chamber (20).

## Revendications

1. Dispositif de production de biogaz de matières organiques, comportant un réacteur à biogaz (10), qui comporte une chambre d'entrée (20) à être remplie avec les matières organiques et une voie de retour (36) pour extraire au moins partiellement les matières organiques du réacteur à biogaz (10), **caractérisé en ce que** le réacteur à biogaz (10) comporte de plus une première chambre intermédiaire (30) et une deuxième chambre intermédiaire (32), la chambre d'entrée (20), la première chambre intermédiaire (30), la deuxième chambre intermédiaire (32) et la voie de retour (36) formant, dans cet ordre, des sections d'une voie d'écoulement pour les matières organiques, la voie d'écoulement ne permettant un écoulement que dans un seul sens, où, parmi deux sections consécutives, l'une constitue une voie d'écoulement montante et l'autre constitue une voie d'écoulement descendante, respectivement, que la chambre d'entrée (20) comporte un bord de débordement (22), au moyen duquel la première chambre intermédiaire (30) peut être remplie avec les matières organiques de la chambre d'entrée (20), que la première chambre intermédiaire (30) et la deuxième chambre intermédiaire (32) communiquent dans une zone au-dessous d'un orifice d'entrée (34) de la voie de retour (36) et au-dessus du bord de débordement (22), et que la première chambre intermédiaire (30) et la deuxième chambre intermédiaire (32) sont formées dans l'intérieur d'un réservoir intérieur (18) en forme de gobelet, qui est agencé dans l'intérieur du réacteur à biogaz (10).

2. Dispositif selon la revendication 1, **caractérisée en ce qu'**une voie de vidange (42) bifurque de la voie de retour (36) de manière à ce que la voie de vidange (42) forme un tuyau communicant avec la voie de retour (36).

3. Dispositif selon la revendication 1 ou 2, **caractérisé en ce que** la section transversale de la chambre d'entrée (20) est rétréci dans la zone du bord de débordement (22).

4. Dispositif selon l'une des revendications précédentes, **caractérisé en ce que** le réacteur à biogaz (10) comporte une section (14) supérieure qui se rétrécit vers le haut et le bord de débordement (22) s'étend si loin dans la section supérieure (14) que la section transversale de la chambre d'entrée (20) est rétréci dans la zone du bord de débordement (22).

5. Dispositif selon l'une des revendications précédentes, **caractérisé en ce que** le réacteur à biogaz (10) comporte une section inférieure (16) qui se rétrécit vers le bas, une espace de ramassage (56) de sédiments étant formée dans la zone inférieure de la section inférieure (16), qui peut être liée optionnellement d'un côté à l'intérieur du réacteur à biogaz (10) et optionnellement de l'autre côté à l'environnement du réacteur a biogaz (10).

6. Dispositif selon l'une des revendications précédentes, **caractérisé en ce qu'**un conduit d'extraction de gaz (64) est raccordé dans zone la supérieure du réacteur à biogaz, par lequel une pression constante peut être appliquée à l'intérieur du réacteur à biogaz (10).

7. Dispositif selon la revendication 6, **caractérisé en ce qu'**en plus un réservoir de liquide (66), qui est remplissable d'un liquide (76), est prévu, le bout (78) du conduit d'extraction de gaz (64) qui mène de l'intérieur du réacteur à biogaz (10) vers l'extérieur peut être placé dans le liquide (76) de sorte que la pression dans l'intérieur du réacteur à biogaz (10) est ajustable par le biais de la profondeur d'immersion dans le liquide (76).

8. Dispositif selon l'une des revendications précédentes, **caractérisé en ce que** de plus une unité de mélange (86) pour remplir la chambre d'entrée (20) est prévue, l'unité de mélange étant adaptée à mélanger des matières organiques nouvelles et des matières organiques extraites du réacteur à biogaz (10) au moyen de la voie de retour (36) dans un rapport qui vaut essentiellement 1:1.

9. Dispositif selon l'une des revendications précédentes, **caractérisé en ce que** de plus un échangeur de chaleur (88) agencé en amont de la chambre d'entrée (20) est prévue, l'échangeur de chaleur (88) étant adapté à préchauffer, au moyen d'un fluide chaud, les matières organiques à introduire.

10. Dispositif selon l'une des revendications précédentes, **caractérisé en ce qu'**une unité de pilage (94) est prévu en amont de la chambre d'entrée (20).

11. Dispositif selon l'une des revendications précédentes, **caractérisé en ce qu'**un réservoir de gaz (152) est prévu, dans lequel du biogaz produit dans le réacteur à biogaz (10) peut être stocké et qui entoure le réacteur a biogaz (10) au moins partiellement.

12. Procédé de production de biogaz, comportant les étapes:
- introduction de matières organiques dans une chambre d'entrée (20) d'un réacteur à biogaz (10);
- transport des matières organiques en sens unique suivant les sections formées par la chambre d'entrée (20), du moins la première chambre intermédiaire (30) et une deuxième chambre intermédiaire (32), qui sont formées dans l'intérieur d'un réservoir intérieur en forme de gobelet agencé dans le réacteur à biogaz (10), et une voie de retour (36), où, parmi deux sections consécutives, l'une constitue une voie d'écoulement montante et l'autre constitue une voie d'écoulement descendante, respectivement;
- dégazage des matières organiques en exposant temporairement les matières organiques lors de la transition de la chambre d'entrée (20) dans la première chambre intermédiaire (30).

13. Procédé selon la revendication 12, **caractérisé en ce que** les étapes du procédé qui ont lieu à l'intérieur du réacteur à biogaz (10) sont effectuées sans agitation active des matières organiques.

14. Procédé selon l'une des revendications 12 ou 13, **caractérisé en ce que** les matières organiques nouvellement introduites sont préchauffées au moyen d'un échangeur de chaleur (88) à 35 °C - 37 °C avant d'être introduites dans la chambre d'entrée (20).

15. Procédé selon l'une des revendications 12 à 14, **caractérisé en ce que** les matières organiques introduites dans la chambre d'entrée sont mélangées de matières organiques préchauffées nouvellement introduites et de matières organiques extraites directement au moyen de la voie de retour (36).

16. Procédé selon la revendication 15, **caractérisé en ce que** le rapport de mélange vaut 1 :1.

17. Procédé selon les revendications 12 à 16, **caractérisé en ce qu'**une pression intérieure est maintenu constante dans l'intérieur du réacteur à biogaz (10).

18. Procédé selon l'une des revendications 12 à 17, **caractérisé en ce que** les matières organiques peuvent être introduites de la chambre d'entrée (20) à travers un bord de débordement (22) de la chambre d'entrée (20) dans la au moins une chambre intermédiaire (30) et les matières organiques sont densifiées dans la zone du bord de débordement (22) par un rétrécissement de la section transversale de la chambre d'entrée (20).
